# EUROPÄISCHE PATENTSCHRIFT

(11) **EP 4 533 183 B1**
(45) Veröffentlichungstag und Bekanntmachung des Hinweises auf die Patenterteilung: **14.10.2026**
(21) Anmeldenummer: 23731162.6
(22) Anmeldetag: 02.06.2023
(51) Int. Cl.: G03H 1/08, G03H 1/04, A61B 1/00, G02B 5/04, G02B 5/18, A61B 1/06, A61B 1/07, G03H 1/02, G03H 1/26

(54) **OPTISCHES SYSTEM ZUR DIGITALEN HOLOGRAFIE**
OPTICAL SYSTEM FOR DIGITAL HOLOGRAPHY
SYSTÈME OPTIQUE POUR HOLOGRAPHIE NUMÉRIQUE

(30) Priorität: 03.06.2022 DE 102022114150
(43) Veröffentlichungstag der Anmeldung: 09.04.2025
(73) Patentinhaber: AKMIRA OPTRONICS GMBH, 14480 Potsdam (DE)
(72) Erfinder: KNÜTTEL, Alexander, 14480 Potsdam (DE)
(74) Vertreter: Schulz Junghans Patentanwälte PartGmbB
(86) Internationale Anmeldenummer: PCT/EP2023/064867
(87) Internationale Veröffentlichungsnummer: WO 2023/233016

(56) Entgegenhaltungen:
- DE-A1- 102015 113 465
- DE-T2- 69 815 361
- US-A1- 2004 070 853
- US-B1- 6 525 846

## Beschreibung

Die Erfindung betrifft ein optisches System zur digitalen Holografie, insbesondere zur Anwendung im minimal-invasiven medizinischen Bereich.

Aus dem Stand der Technik sind sogenannte GRISMs als optische Komponenten bekannt, die aus einer Kombination aus einem optischen Gitter und einem Prisma bestehen (aus dem englischen: GRating + pRISM). Bei einem GRISM propagiert Licht der Zentralwellenlänge geradeaus durch das GRISM, und spaltet das Licht trotzdem spektral auf. Damit kann in besonders einfacher Weise ein spektral bildgebendes Gerät aufgebaut werden.

Zudem sind im Bereich der digitalen Holografie verschiedene Systeme bekannt, bei denen ein kohärenter Referenzlichtstrahl mit einem kohärenten Objektlichtstrahl überlagert wird, wobei aus dem Interferenzmuster der Überlagerung die räumliche Struktur einer Oberfläche eines Objekts, von der das Objektlicht reflektiert oder zurückgestreut wurde, bevor es zur Überlagerung mit dem Referenzlicht gebracht wurde, berechnet werden kann. Dazu weisen solche Systeme eine Kamera auf, die dazu eingerichtet ist, das Interferenzmuster aufzunehmen. Als Quelle für das Referenzlicht und das Objektlicht wird typischer Weise ein Laser verwendet, dessen Laserlicht mittels eine Strahlteilers in den Referenzlichtstrahl und den Objektlichtstrahl aufgeteilt wird. So ist die räumliche und zeitliche Kohärenz von Referenzlicht und Objektlicht gewährleistet. Systeme dieser Art werden bevorzugt in bildgebenden 3D-endoskopischen Verfahren im Bereich der minimal-invasiven Medizin eingesetzt.

Aus der US 6,525,846 B1 ist ein Wellenlängen-Multiplexer bekannt, der ein Volumen-Phasen-Gitter, sowie zwei gegenüberliegende reflektierende Seiten für eingekoppeltes Licht aufweist. Der Wellenlängenmultiplexer ist zum Strahlteilen und Wellenlängensplitting eingerichtet. Zur digitalen Holografie eignet sich diese Vorrichtung nicht.

Die DE 698 15 361 T2 offenbart eine Vorrichtung zum Bilden von wenigstens ersten und zweiten farbigen, unter einem Winkel getrennten Lichtstrahlen bekannt. Die Vorrichtung ist nicht dazu eingerichtet, Objekt- und Referenzstrahlen über ein transmissionsholografisches Verfahren zu kombinieren, sondern erlaubt lediglich die spektrale Auftrennung eines einfallenden Lichtstrahls mittels einer Reflexionsgitteranordnung.

Ausgehend von dem bekannten Stand der Technik soll ein verbessertes System geschaffen werden, das eine multi-spektrale digitale Holografie insbesondere im Bereich der minimal-invasiven Medizintechnik ermöglicht.

Diese Aufgabe wird durch ein optisches System zur digitalen Holografie gelöst, das zumindest die folgenden Komponenten aufweist:
Eine erste Holografie-Anordnung, umfassend ein erstes Beugungselement, welches durch eine erste Prisma-Anordnung mit viereckiger Grundfläche gebildet ist, wobei eine Mantelfläche der ersten Prisma-Anordnung die folgenden Mantelflächenbereiche aufweist:
a) eine erste, insbesondere planare Eintrittsfläche für Referenzlicht, die sich entlang einer ersten Eintrittsebene erstreckt, wobei die erste Eintrittsfläche Teil einer ersten Stirnfläche sein kann und
b) eine zweite, insbesondere planare Eintrittsfläche für Objektlicht, die sich entlang einer zweiten Eintrittsebene erstreckt, wobei die erste und die zweite Eintrittsfläche gegenüberliegende Mantelflächenbereiche der ersten Prisma-Anordnung ausbilden, wobei die zweite Eintrittsfläche Teil einer zweiten Stirnfläche sein kann
c) eine insbesondere planare Austrittsfläche, die sich entlang einer Austrittebene erstreckt und durch die gebeugtes Referenzlicht und gebeugtes Objektlicht aus dem ersten Beugungselement austreten kann,
d) eine der Austrittsfläche gegenüberliegende Prismafläche,
e) eine optische Transmissions-Beugungsgitteranordnung, die im ersten Beugungselement angeordnet ist und sich entlang einer Beugungsebene, welche die erste Eintrittsebene und die Austrittsebene schneidet, zwischen der ersten Eintrittsfläche und insbesondere der Austrittsfläche erstreckt, insbesondere so dass die Transmissions-Beugungsgitteranordnung mit der ersten Eintrittsfläche und der Austrittfläche ein Dreiecksprisma bilden.

Die Erfindung sieht weiterhin vor, dass die Transmissions-Beugungsgitteranordnung des ersten Beugungselements zumindest ein erstes Volumen-Phasen-Hologramm Gitter umfasst, und dass die erste Holografie-Anordnung auf Seite der Prismafläche des ersten Beugungselements einen ersten insbesondere planaren Spiegel mit einer ersten Spiegelebene aufweist, wobei die erste Spiegelebene mit der Beugungsebene einen Winkel α einschließt, und die Prismafläche einen Winkel ω₂ mit der Beugungsebene einschließt, wobei mindestens einer oder beide Winkel α, ω₂ ungleich 45° beträgt/betragen, insbesondere von 45° um mehr als ± 0.2° abweichen, insbesondere wobei der Winkel α = 45° ± β ist mit β > 0°, insbesondere wobei der Winkel ω₂ = 45° ± ρ₂ ist mit ρ₂ > 0°, insbesondere um einen maximal möglichen Wirkungsgrad für Objekt- und Referenzlicht in der ersten Holografie-Anordnung zu erzielen.

Bevorzugt sind β und/oder ρ₂ größer als 0.2.

Bevorzugt sind β und/oder ρ₂ kleiner als 20°.

Gemäß der Erfindung sind die Winkel β bzw. ρ₂ insbesondere so gewählt, dass auf die Transmissions-Beugungsgitteranordnung unter einem Winkel von 45° auftreffendes Referenzlicht von der Transmissions-Beugungsgitteranordnung in Richtung Prismafläche gebeugt wird und von dem ersten Spiegel so reflektiert wird, dass das reflektierte Referenzlicht aufgrund eines Einfallswinkels des reflektierten Referenzlichtes auf die Transmissions-Beugungsgitteranordnung zu einem geringeren Anteil, insbesondere einen um mindestens 30% geringeren Anteil, als das ursprünglich von Seite der ersten Eintrittsfläche auf die Transmissions-Beugungsgitteranordnung treffendes Referenzlicht wieder in Richtung erster Eintrittsfläche gebeugt wird und ein verbleibender Anteil des reflektierten Referenzlichtes durch die Transmissions-Beugungsgitteranordnung in Richtung Austrittsfläche propagiert.

Die Begriffe ,Referenzlicht' und 'Objektlicht' sind im Zusammenhang mit digitaler Holografie aus dem Stand der Technik bekannt. Dabei wird das Referenzlicht mit dem vom Objekt zurückgeworfenen (z.B. reflektierten oder gestreuten) Objektlicht, das insbesondere aus der gleichen kohärenten Lichtquelle stammt wie das Referenzlicht, im Beugungselement überlagert, so dass nach weiterer Propagation ein Interferenzmuster nahe der Austrittsfläche an einem Array-Detektor, wie beispielsweise einem 2D Sensor oder einer Kamera entsteht. Dieses Muster beinhaltet insbesondere die vollständige Welleninformation über das Objektlicht, so dass aus dem Interferenzmuster das Objekt in Form eines Hologramms berechnet werden kann. Insbesondere lässt sich so eine Tiefeninformation, also eine 3D-Information über das Objekt generieren, insbesondere wenn eine Vielzahl an Wellenlängen, beispielsweise in Form spektralen Linien für das Referenzlicht und Objektlicht eingesetzt werden.

Die Erfindung lässt sich daher besonders gut im Zusammenhang mit minimal-invasiven medizinischen bildgebenden Instrumenten kombinieren, die dazu ausgebildet sein sollen, eine 3D-Information aus dem Sichtbereich des Instruments zu erzeugen. Stereoskopische Optiken sind für den minimal-invasiven Einsatz zu groß, wodurch digitale Holografie in diesem Bereich der Technik von großem Nutzen ist.

Im erfindungsgemäßen Zustand ist insbesondere vorgesehen, dass Referenzlicht über die erste Eintrittsfläche, insbesondere kollimiert in das Beugungselement eintritt. Dort wird es durch die Transmissions-Beugungsgitteranordnung in Richtung Prismafläche gebeugt. Durch den Spiegel wird das gebeugte Referenzlicht in Richtung der Transmissions-Beugungsgitteranordnung zurückreflektiert - allerdings unter einem Winkel, der sich aus dem Snell'schen Gesetz berechnen lässt, durch den Winkel wird zumindest ein Teil des reflektierten Referenzlichtes von der Transmissions-Beugungsgitteranordnung nicht gebeugt, sondern transmittiert und propagiert in Richtung der Austrittsfläche. Das Objektlicht tritt erfindungsgemäß über die zweite Eintrittsfläche in das Beugungselement ein, und wird durch die Transmissions-Beugungsgitteranordnung direkt in Richtung Austrittsfläche gebeugt, wo es mit dem Referenzlicht auf einem Array-Detektor bzw. Kamerachip zur Überlagerung und insbesondere Interferenz kommt.

Die Erfindung stellt ein optisches System zur digitalen Holografie zur Verfügung, das es ermöglicht, multiple Wellenlängen, die im Referenzlicht umfasst sind, insbesondere wobei die Wellenlängen in Form von spektral beabstandeten spektralen Linien vorliegen, aus einem vergleichsweise großen Wellenlängenbereich auf Seite der Austrittsfläche zu separieren, und welches gleichzeitig durch eine äußerst kompakte und robuste Bauweise besticht.

Insbesondere werden in äußerst vorteilhafter Weise die optischen Eigenschaften eines Volumen-Phasen-Hologramm Gitters mit einem in Relation zur Transmissions-Beugungsgitteranordnung vorteilhaft geneigten ersten Spiegel kombiniert, die es erst ermöglicht, die Bandbreite an Wellenlängen aufzulösen.

Durch den erfindungsgemäß angeordneten ersten Spiegel kann eine spätere Bildinformation, die aus dem mit dem Referenzlicht überlagerten Objektlicht an der Austrittsfläche gewonnen wird, außerhalb des typischer Weise störenden DC-Bereichs geschoben werden.

Weiterhin wird durch den ersten Spiegel erreicht, dass der am ersten Spiegel reflektierte Teil des Referenzlichtes beim Durchgang durch die erste Transmissions-Beugungsgitteranordnung nur leicht - oder gar nicht - durch Transmissions-Beugungsgitteranordnung abgeschwächt wird. Würde der Winkel β und ρ₂ gleich 0° betragen, dann würde ein sehr großer Teil des Referenzlichtes durch die Transmissions-Beugungsgitteranordnung wieder zurück zur ersten Eintrittsfläche gebeugt werden und somit nicht zur Austrittsfläche gelangen.

Der erste Spiegel umfasst eine erste Spiegelebene, die sich entlang einer reflektierenden Schicht des ersten Spiegels erstreckt, insbesondere wobei der erste Spiegel aus der reflektierenden Schicht besteht.

Im Folgenden ist unter einem Winkel des ersten Spiegels bezüglich einer Fläche oder einer Ebene stets der Winkel der ersten Spiegelebene des ersten Spiegels mit der Fläche oder der entsprechenden Ebene gemeint.

Der Ausdruck ,Prisma' im Kontext dieser Erfindung bezieht sich insbesondere auf einen geometrischen, insbesondere physikalischen Körper, der zwei sich gegenüberliegende Grundflächen aufweist, die über an den Kanten der Grundflächen angeordnete Mantelflächenbereiche einer Mantelfläche miteinander verbunden sind.

Erfindungsgemäß ist die Grundfläche der Prisma-Anordnung zumindest viereckig, d.h. die Grundfläche weist ein Polygon auf, wobei das Polygon mindestens vier Ecken hat. Es ist allerdings ohne Weiteres möglich und denkbar, dass die Grundfläche weitere Ecken umfasst und damit weitere Mantelflächenbereiche, die beispielsweise zu anderen Zwecken ausgebildet sind von der Prisma-Anordnung umfasst sind.

Ein viereckiges Prisma weist demnach vier Mantelflächenbereiche auf, die zwei viereckige Grundflächen begrenzen. Sofern nicht anders definiert, haben die Grundflächen eine identische Form und Größe.

Der Begriff ,Prisma-Anordnung' im Kontext der vorliegenden Erfindung bezieht sich insbesondere auf eine feststehende Anordnung von mindestens einem Prisma oder mehreren Prismen.

Gemäß einer Ausführungsform der Erfindung ist die zumindest viereckige Prisma-Anordnung integral bzw. monolithisch ausgeformt.

Die Begriffe 'integral ausgebildet' oder ,integral ausgeformt' sind im Kontext der Erfindung synonym zu ,monolithisch' zu verstehen.

Gemäß einer Ausführungsform der Erfindung ist die zumindest viereckige Prisma-Anordnung durch ein erstes und ein zweite Dreiecksprisma gebildet, wobei die beiden Dreiecksprismen entlang der Beugungsebene über die Transmissions-Beugungsgitteranordnung miteinander verbunden sind. Das erstes Dreiecksprisma der zwei Dreiecksprismen umfasst dabei insbesondere die erste Eintrittsfläche, die Austrittfläche. Das zweites Dreiecksprisma der zwei Dreiecksprismen umfasst dabei insbesondere die zweite Eintrittsfläche, die Prismafläche. Insbesondere ist die viereckige Prisma-Anordnung aus zwei separaten fest miteinander verbundenen Dreiecksprismen gebildet.

Allgemein gilt, dass die zumindest viereckige Prisma-Anordnung bei jeder Ausführungsform der Erfindung monolithisch ausgeführt sein kann - sofern nicht explizit anders offenbart. Alternativ kann die zumindest viereckige Prisma-Anordnung in analoger Weise durch verschiedene miteinander verbundene Prismen gebildet werden.

Wenn keine Angaben über die Zusammensetzung und/oder die Natur der Prisma-Anordnung gemacht werden, so sind sowohl die monolithische Ausführung als auch Ausführungsformen umfasst, bei denen die Prisma-Anordnung beispielsweise durch eine Vielzahl separater miteinander verbundener Prismen gebildet ist.

Insbesondere sind alle Mantelflächenbereiche der zumindest viereckigen Prisma-Anordnung planar.

Gemäß einer Ausführungsform der Erfindung umfasst die Prisma-Anordnung einen massiven transparenten Körper, der insbesondere ein transparentes Glas oder Polymer umfasst.

Gemäß einer Ausführungsform der Erfindung besteht die Prisma-Anordnung insbesondere mit Ausnahme der Transmissions-Beugungsgitteranordnung aus einem transparenten Glas und/oder Polymer.

Gemäß einer Ausführungsform der Erfindung weist der erste Spiegel einen Reflexionsgrad zwischen 10% und 100%, insbesondere mehr als 70% auf. Der Reflexionsgrad kann bis nahe 100% betragen, also beispielsweise 95% oder sogar 99%.

Da typischer Weise (im Vergleich zum Objektlicht) ausreichend Referenzlicht zur Verfügung steht, muss die Reflexionsgrad des ersten Spiegels nicht besonders hoch sein.

Der erste Spiegel weist insbesondere eine Glattheit von gleich oder besser als λ/2 auf.

Gemäß einer weiteren Ausführungsform der Erfindung ist die Transmissions-Beugungsgitteranordnung eine im wesentliche planare Transmissions-Beugungsgitteranordnung.

Volumen-Phasen-Hologramm (Transmissions) Gitter (im Englischen "Volume Phase Holographic (Transmission) Grating") oder abgekürzt VPH sind aus dem Stand der Technik bekannt. Das Volumen-Phasen-Hologramm Gitter ist insbesondere ein Transmissionsgitter. Volumen-Phasen-Hologramm Gitter bestehen beispielsweise aus einem Film aus dichromierter/dichromatischer Gelatine (DCG) zwischen zwei Glassubstraten, insbesondere wobei die Glassubstrate durch das erste und das zweite Dreiecksprisma gebildet werden, insbesondere wobei die Gelatine zwischen Mantelflächenbereichen des ersten und des zweiten Dreiecksprismas angeordnet ist, die sich entlang der Beugungsebene erstecken. Volumen-Phasen-Hologramm Gitter bieten eine hohe Beugungsspitzeneffizienz erster Ordnung, und eine gleichmäßige Leistung über eine vorgegebene Bandbreite.

Alternativ zu Gelatine kann auch ein Photopolymer verwendet werden, welches durch entsprechende Belichtung zu einem Volumen-Phasen-Hologramm Gitter ausgebildet wird. Insbesondere kann das Photopolymer nach Belichtung zwischen die zwei Glassubstrate eingebracht werden. Photopolymere haben gegenüber Gelatine den Vorteil, nicht zähflüssig und damit leichter handhabbar zu sein. Sowohl für Gelatine als auch Photopolymere sind Schichtdicken für das Volumen-Phasen-Hologramm Gitter in der Größenordnung von wenigen Mikrometern bis etwa 100 µm möglich.

Des Weiteren sind auch Volumen-Phasen-Hologramm Gitter bekannt, die in Glas eingebracht sind, und insbesondere keine Gelatine aufweisen.

Volumen-Phasen-Hologramm Gitter in PTR-Glas haben gegenüber anderen Volumen-Phasen-Hologramm Gitter aus Gelatine oder Photopolymer den Vorteil, dass diese thermisch stabiler sind, da dies typische Eigenschaften von Glas sind. Da die Brechungsindex-Modulationen aber in PTR-Glas etwa eine Größenordnung schwächer sind als in Gelatine und Photopolymeren, muss die aktive Glasschicht entsprechend dicker sein. Dies führt aber zu Einengungen in der Winkel-Dispersion bzw. in der Spektral-Charakteristik. D.h. je nach Anforderung an das Beugungselement, kann ein entsprechendes Volumen-Phasen-Hologramm Gitter Material und Herstellungsverfahren gewählt werden.

Insbesondere ist die erste Holografie-Anordnung dazu eingerichtet und ausgebildet, in einer sogenannten "Littrow-Anordnung" betrieben zu werden. Dabei wird das Beugungselement und damit die erste Holografie-Anordnung so im System angeordnet, dass die Beugungsebene einen Winkel von 45° mit einer optischen Achse des Beugungselements einschließt und damit entsprechend die erste Spiegelebene den Winkel β und/oder die Prismafläche einen Winkel ρ₂ mit der optischen Achse einschließt. Dadurch wird das über die erste Eintrittsfläche in das erste Beugungselement eingetretene Referenzlicht, das zunächst entlang der optischen Achse propagiert, von der Transmissions-Beugungsgitteranordnung idealerweise um einen Beugungswinkel von 2 · 45° = 90° gebeugt, insbesondere wobei das auf die Transmissions-Beugungsgitteranordnung auftreffende Referenzlicht einen Winkel von 45° mit der Beugungsebene einschließt und das gebeugte Referenzlicht ebenfalls einen Winkel von 45° mit der Beugungsebene einschließt. Das gebeugte Referenzlicht wird sodann von dem ersten Spiegel, reflektiert, wobei der Winkel der Spiegelebene α und/oder der Winkel Prismaebene ω₂ dazu beitragen, dass das reflektierte Referenzlicht unter einem von 45° verschiedenen Winkel auf die Transmissions-Beugungsgitteranordnung trifft und deswegen zu einem großen Teil durch die Transmissions-Beugungsgitteranordnung propagiert ohne gebeugt zu werden.

Diese Funktionalität kann wie erfindungsgemäß beansprucht, dadurch erreicht werden, dass der erste Spiegel und/oder die Prismafläche in einem von 45° verschiedenen Winkel zu Beugungsebene angeordnet sind.

Auf diese Weise kann das erfindungsgemäße System besonders vorteilhaft bei digitalen Holografie Anwendungen eingesetzt werden.

Es ist festzuhalten, dass die Littrow-Anordnung, also eine Beugung um 90° lediglich für eine Zentralwellenlänge für ein optisches Gitter möglich ist. Davon abweichende Wellenlängen, werden entsprechend der Winkel-Dispersion um einen anderen Winkelbetrag gebeugt.

Auch die Beugung von 90° für die Zentralwellenlänge kann etwas variieren, wenn die Transmissions-Beugungsgitteranordnung entweder nicht exakt im 45° Winkel zur optischen Achse steht, oder die Gitterfrequenz nicht ganz der Littrow-Bedingung entspricht.

Gemäß einer weiteren Ausführungsform der Erfindung, ist das mindestens eine erste Volumen-Phasen-Hologramm Gitter der Transmissions-Beugungsgitteranordnung so eingerichtet, dass es eine erste Bandbreite λₒ ± Δλ an Wellenlängen um eine Zentralwellenlänge λ₀ in einem Beugungswinkelbereich um 90° ± Δ*θ* beugt, wenn es in einer Littrow-Anordnung betrieben wird.

Die optische Achse des ersten Beugungselements kann von der ersten bzw. von der zweiten Eintrittsfläche kommend in Richtung Beugungsebene zeigen und die Beugungsebene in einem 45° Winkel schneiden. Sofern nicht explizit anders gefordert, wird davon ausgegangen, dass im erfindungsgemäßen Zustand des Systems, Referenzlicht und Objektlicht jeweils entlang der optischen Achse auf die Beugungsebene treffen.

Gemäß der Erfindung kann jeder Fläche, also der ersten und zweiten Eintrittsfläche, der Austrittsfläche und der Prismafläche eine Ebene zugeordnet werden, die sich entlang der jeweiligen Fläche erstreckt. Wenn auf einen Winkel zwischen zwei Flächen Bezug genommen wird, so ist damit auch der Winkel zwischen den Ebenen gemeint. Die Winkelangabe beziehen sich sofern nicht explizit anders offenbart auf die innenliegenden Winkel der Prisma-Anordnung.

Insbesondere ist die erste Eintrittsfläche durch eine Kante mit der Austrittsfläche einerseits und durch eine imaginäre Schnittlinie der Beugungsebene mit der ersten Eintrittsebene andererseits begrenzt.

Insbesondere ist die Austrittsfläche durch eine Kante mit der ersten Eintrittsfläche einerseits und durch die imaginäre Schnittlinie der Beugungsebene mit der Austrittsebene und/oder durch die Kante mit der zweiten Eintrittsfläche andererseits begrenzt.

Weiterhin sind Richtungsangaben, Orientierungen und Positionen im Kontext der Erfindung insbesondere für einen optisch entfalteten Zustand des Systems zu verstehen. Dem Fachmann ist klar, dass zum Beispiel die Richtung einer optischen Achse durch einen Umlenkspiegel im System geändert werden kann, wobei im optisch-funktionalen Sinn, keine Richtungsänderung stattfindet, sondern lediglich im geometrischen Sinn. Dieses Verständnis ist auch zur Auslegung der Ansprüche heranzuziehen.

Gemäß einer Ausführungsform der Erfindung, schließt die Beugungsebene des ersten Beugungselements eine Winkel ω₁ mit der ersten Eintrittsfläche des ersten Beugungselements ein, insbesondere wobei der Winkel 30° ≤ ω₁ ≤ 60 °, insbesondere wobei ω₁ = 45°.

Gemäß einer Ausführungsform der Erfindung, schließt die Beugungsebene des ersten Beugungselements einen Winkel ω₂ mit der Prismafläche des ersten Beugungselements ein, insbesondere wobei der Winkel 30° ≤ ω₂ ≤ 60 °, insbesondere wobei ω₂ = 45° oder ω₂ = 45° ± β, insbesondere wobei ω₂ so gewählt ist, dass die Prismafläche mit der Spiegelebene des ersten Spiegels parallel verläuft.

Gemäß einer Ausführungsform der Erfindung, schließt die Beugungsebene des ersten Beugungselements einen Winkel ω₃ mit der zweiten Eintrittsfläche des ersten Beugungselements ein, insbesondere wobei der Winkel 30° ≤ ω₃ ≤ 60 °, insbesondere wobei ω₃ = 45°.

Gemäß einer Ausführungsform der Erfindung, schließt die Beugungsebene des ersten Beugungselements einen Winkel ω₄ mit der Austrittsfläche des ersten Beugungselements ein, insbesondere wobei der Winkel 30° ≤ ω₄ ≤ 60 °, insbesondere wobei ω₄ = 45° oder ω₄ = 45° ± β, insbesondere wobei ω₄ so gewählt ist, dass die Austrittsebene mit der Spiegelebene des ersten Spiegels parallel verläuft.

Gemäß einer Ausführungsform der Erfindung sind die erste und die zweite Eintrittsfläche des ersten Beugungselements parallel zueinander ausgerichtet. Gemäß einer weiteren Ausführungsform der Erfindung, betragen die Winkel ω₁ und ω₃ gleich 45°. In dieser Konfiguration ist es insbesondere möglich Referenzlicht und Objektlicht senkrecht auf die erste und zweite Eintrittsfläche zu strahlen, da die Eintrittsflächen rechtwinklig auf die optische Achse des Beugungselements stehen. Gemäß einer Ausführungsform der Erfindung sind die Prismafläche und die Austrittfläche des ersten Beugungselements parallel zueinander ausgerichtet. Gemäß der Erfindung umfasst das System einen ersten optischen Array-Detektor, wie beispielsweise eine Kamera, wobei der erste Array-Detektor dazu eingerichtet ist, aus der Austrittsfläche des ersten Beugungselements austretendes Licht zu detektieren.

Insbesondere ist der erste Array-Detektor auf Seite der Austrittfläche des ersten Beugungselements der ersten Holografie-Anordnung angeordnet.

Weiterhin kann vorgesehen sein, dass der erste Array-Detektor auf der Austrittfläche des ersten Beugungselements der ersten Holografie-Anordnung angeordnet ist und insbesondere wobei der erste Array Detektor mit der Austrittsfläche des ersten Beugungselements verbunden ist, so dass zwischen Austrittsfläche und Array-Detektor zumindest kein Luftspalt ist.

Alternativ kann der erste Array-Detektor zu der Austrittsfläche beabstandet sein, und einen Luftspalt zwischen Austrittsfläche und Array-Detektor umfassen.

Weiterhin kann der erste Array-Detektor dazu eingerichtet sein, das detektierte Licht in ein elektrisches Signal umzuwandeln, das von einer Auswerteeinrichtung des Systems zumindest hinsichtlich einer zweidimensionalen Intensitätsverteilung des detektierten Lichts auf dem ersten Array-Detektor ausgewertet werden kann.

Durch den Array-Detektor kann eine Detektionsebene gegeben sein, die sich entlang dem Array des Array-Detektors erstreckt, also beispielsweise entlang einer Pixelmatrix der Kamera.

Gemäß einer weiteren Ausführungsform der Erfindung ist vorgesehen, dass die Detektionsebene des ersten Array-Detektors mit der Beugungsebene einen Winkel von ε = 45° ± η mit η>0° einschließt, insbesondere wobei η = β, insbesondere wobei die Detektionsebene parallel zum ersten Spiegel verläuft. Weiterhin kann die Austrittsebene parallel oder ko-planar zur Detektionsebene verlaufen.

Insbesondere ist 0°< η < 20°.

Gemäß einer Ausführungsform der Erfindung kann vorgesehen sein, dass die Detektionsebene eine solchen Winkel mit der Beugungsebene einschließt, dass von der Detektionsebene reflektiertes Licht unter einer Littrow-Geometrie auf die Beugungsebene trifft, so dass das reflektierte Licht von der Transmissions-Beugungsgitteranordnung vollständig oder zumindest größtenteils in Richtung der zweiten Eintrittsfläche gebeugt wird und dort austreten kann. Somit kann eine durch mehrfach reflektiertes Licht entstehendes "ghosting"-Signal auf dem Array-Detektor vermieden werden.

Die vorerwähnte Ausbildungsformen der Erfindung hat den Vorteil, dass verhindert werden kann, dass vom Array-Detektor rückreflektiertes Licht abermals zum ersten Spiegel gelangt und von dort wiederum auf den Array-Detektor reflektiert wird und dort ein ungewolltes Signal erzeugt. Durch Wahl des Winkels η, kann das vom Array-Detektor reflektierte Licht vollständig oder größtenteils von der ersten Transmissions-Beugungsgitteranordnung in Richtung der zweiten Eintrittsfläche gebeugt werden.

Gemäß einer weiteren Ausführungsform der Erfindung ist vorgesehen, dass die Grundfläche der ersten Prisma-Anordnung ein Parallelogramm bildet, insbesondere wobei die Beugungsebene des ersten Beugungselements entlang einer Diagonalen verläuft, die sich in der Grundfläche zwischen der ersten Eintrittsfläche und der Austrittsfläche des ersten Beugungselements erstreckt, insbesondere wobei
i) die erste Eintrittsfläche mit der Beugungsebene des ersten Beugungselements einen Winkel ω₁ = 45°± ρ₁ einschließt und die Prismafläche mit der Beugungsebene des ersten Beugungselements einen Winkel ω₂ = 45° einschließt, wobei ρ₁ > 0°, insbesondere wobei ρ₁ > 0.2°, oder
ii) die erste Eintrittsfläche mit der Beugungsebene des ersten Beugungselements einen Winkel ω₁ = 45° einschließt und die Prismafläche mit der Beugungsebene des ersten Beugungselements einen Winkel ω₂ = 45°± ρ₂ einschließt, wobei ρ₂ > 0°, insbesondere wobei ρ₂ > 0.2°, oder
iii) die erste Eintrittsfläche mit der Beugungsebene des ersten Beugungselements einen Winkel ω₁ = 45°± ρ₁ einschließt und die Prismafläche mit der Beugungsebene des ersten Beugungselements einen Winkel ω₂ = 45°± ρ₂ einschließt, wobei ρ₁ > 0° und ρ₂ > 0°, insbesondere wobei ρ₁ > 0.2° und ρ₂ > 0.2°, oder
iv) die erste Eintrittsfläche mit der Beugungsebene des ersten Beugungselements einen Winkel ω₁ = 45° einschließt und die Prismafläche mit der Beugungsebene des ersten Beugungselements einen Winkel ω₂ = 45°einschließt, die Grundfläche ein spezielles Parallelogramm ist, nämlich ein Rechteck, vorzugsweise ein Quadrat, insbesondere wobei die Beugungsebene des ersten Beugungselements entlang einer Diagonalen verläuft, die sich in der Grundfläche zwischen der ersten Eintrittsfläche und der Austrittsfläche des ersten Beugungselements erstreckt.

Jede der Ausführungsformen i) bis iii) reduziert potenzielle Störreflexe, die beim Ein- oder Austritt von Referenz und/oder Objektlicht in die Prisma-Anordnung entstehen, in vorteilhafter Weise.

Besonders die Ausführungsformen ii) und iii) ermöglichen, dass der erste Spiegel direkt auf der Prismafläche angeordnet wird, wenn beispielsweise der Winkel ρ₂ = β gewählt ist.

Weiterhin kann in den Ausführungsformen ii) und iii) der erste Array-Detektor ebenfalls direkt auf der Austrittfläche angeordnet sein, wie oben bereits erwähnt.

Dabei kommen die Vorteile einer reflexarmen Geometrie und die zuvor zum Array-Detektor aufgeführten Vorteile bezgl. einer Rückreflexion vom Array Detektor in Littrow-Konfiguration besonders zum Tragen, da der Winkel zwischen Detektionsebene und Beugungsebene ε = 45° ± η mit η = β = ρ₂ einschließt, so dass die Detektionsebene parallel zum ersten Spiegel verläuft. Weiterhin verläuft die Austrittsebene im Wesentlichen ko-planar zur Detektionsebene.

Insbesondere, wenn zumindest die zweite Eintrittsfläche entspiegelt ist, ist der an der zweiten Eintrittsfläche wieder zurückreflektierte Anteil des Referenzlichtes (der vom ersten Array-Detektor reflektiert und über die Transmissions-Beugungsgitteranordnung gebeugt wurde) zu vernachlässigen. Je nach Strahl-Konfiguration kann es aber auch einen Schrägstellungswinkel an der Austrittsfläche geben (z.B. Ausführungsform iii), so dass eine zusätzliche Rückreflexunterdrückung erreicht wird.

Die Ausführungsformen i) und iii) erlauben in vorteilhafter Weise gleichzeitig eine Polarisationsselektion unter Ausnutzung des Brewsterwinkels und ggf. polarisationsdrehenden Elementen, wie einer λ/2 Platte, durchzuführen und/ oder eine Reduktion von Reflexionen zu erreichen. Referenzlicht und Objektlicht sollten dann unter einem Winkel (also ungleich 90°) so in das erste Beugungselement gekoppelt werden, dass das Referenzlicht und das Objektlicht um die Zentralwellenlänge in einem Winkel von 45° auf die Transmissions-Beugungsgitteranordnung auftreffen, also in Littrow-Konfiguration.

Gemäß der Ausführungsform iv), ist die Grundfläche der ersten Prisma-Anordnung rechteckig, insbesondere quadratisch ausgebildet.

Die Ausführungsform iv) stellt ein einfach herzustellendes erstes Beugungselement zur Verfügung. In dieser Konfiguration kann sowohl das Referenzlicht als auch das Objektlicht senkrecht zur jeweiligen Eintrittsfläche eingestrahlt werden und trifft dadurch in der Littrow-Konfiguration auf die Transmissions-Beugungsgitteranordnung. Weiterhin ist ein einfacher Weise der Winkel α des ersten Spiegels und ggf der Winkel des Array-Detektors einstellbar.

Gemäß einer weiteren Ausführungsform der Erfindung ist der erste Spiegel auf der Prismafläche der ersten Holografie-Anordnung angeordnet.

Diese Ausführungsform der Erfindung umfasst die Möglichkeit, dass der erste Spiegel als reflektierende Schicht auf der Prismafläche ausgebildet ist. Die reflektierende Schicht kann durch bekannte Verfahren auf der Prismafläche erzeugt werden.

Diese Ausführungsform ist eine besonders robuste und kompakte Ausführungsform der Erfindung, da der erste Spiegel direkt mit dem Beugungselement verbunden ist und somit keine Montage oder Justage des Spiegels notwendig ist. Impliziter Weise schließt die Prismafläche dann den Winkel α mit der Beugungsebene ein.

Gemäß einer weiteren Ausführungsform der Erfindung, ist der Winkel β über den ersten Spiegel einstellbar oder eingestellt.

Gemäß einer weiteren Ausführungsform der Erfindung, ist der erste Spiegel um zumindest eine Achse kippbar, so dass der Winkel β über den ersten Spiegel einstellbar oder eingestellt ist.

Im Zusammenhang mit dieser Ausführungsform ist der erste Spiegel insbesondere als separates Element ausgeführt, das optional so ausgebildet ist, dass der Winkel β einstellbar ist.

Gemäß einer weiteren Ausführungsform der Erfindung, umfasst die optische Transmissions-Beugungsgitteranordnung ein zweites Volumen-Phasen-Hologramm Gitter, das sich insbesondere ebenfalls entlang bzw. auf der Beugungsebene erstreckt, wobei das erste und das zweite Volumen-Phasen-Hologramm Gitter eine maximale Beugungsintensität, insbesondere für die erste Beugungsordnung, bei verschiedene Zentralwellenlängen aufweisen, insbesondere wobei Zentralwellenlängen einen Abstand von mindestens der Bandbreite des ersten und des zweiten Volumen-Phasen-Hologramm Gitter aufweisen. So kann insbesondere gewährt werden, dass eine ungestörte spektrale Auftrennung der beiden Zentralwellenlängen an der Austrittsfläche gewährleistet ist.

Diese Ausführungsform erlaubt es im Vergleich zu einem einzigen Volumen-Phasen-Hologramm Gitter einen vergrößerten Spektralbereich für das System nutzbar zu machen, d.h. es können mehrere verschiedene Wellenlängen insbesondere spektrale Linien über das erste Beugungselement spektral an der Austrittsfläche aufgetrennt werden, ohne dass das erste Beugungselement mehr Bauraum benötigt. So könnten beispielsweise über das erste Volumen-Phasen-Hologramm Gitter die Zentralwellenlänge des ersten Volumen-Phasen-Hologramm Gitter und fünf spektralLinien aus der Bandbreite des Volumen-Phasen-Hologramm Gitters gebeugt werden, und über das zweite Volumen-Phasen-Hologramm Gitter könnten neben der Zentralwellenlänge des zweiten Volumen-Phasen-Hologramm Gitters weitere fünf Spektrallinien innerhalb der Bandbreite des zweiten Volumen-Phasen-Hologramm Gitters gebeugt werden.

Gemäß einer Ausführungsform der Erfindung weist das erste Volumen-Phasen-Hologramm Gitter beispielsweise eine Zentralwellenlänge von 600 nm und eine Bandbreite von 10 nm auf.

Gemäß einer Ausführungsform der Erfindung weist das zweite Volumen-Phasen-Hologramm Gitter beispielsweise eine Zentralwellenlänge von 620 nm und eine Bandbreite von 10 nm auf.

Gemäß dieser beiden Ausführungsformen überlappen die Wellenlängenbereiche des ersten und des zweiten Volumen-Phasen-Hologramm Gitters nicht, da die Zentralwellenlängen 20 nm voneinander getrennt sind, was der Summe der Bandbreiten des ersten und des zweiten Volumen-Phasen-Hologramm Gitters entspricht. Je größer der Abstand der Zentralwellenlängen innerhalb der Bandbreiten ist, desto weniger Wechselwirkungen sind zu erwarten.

Gemäß einer weiteren Ausführungsform der Erfindung umfasst die Transmissions-Beugungsgitteranordnung mehr als zwei Volumen-Phasen-Hologramm Gitter, insbesondere wobei die Volumen-Phasen-Hologramm Gitter unterschiedliche Zentralwellenlängen haben. Die Zentralwellenlängen können nach dem oben ausgeführten Prinzip gemäß der Bandbreiten oder mehr spektral beabstandet sein.

Gemäß einer Ausführungsform der Erfindung weist das optische System eine zweite Holografie-Anordnung auf, die ein zweites Beugungselement umfasst, welches durch eine zweite Prisma-Anordnung mit zumindest viereckiger Grundfläche gebildet ist, insbesondere wobei die erste und die zweite Holografie-Anordnung nebeneinander entlang einer optischen Achse des ersten Beugungselements angeordnet sind, wobei eine Mantelfläche der zweiten Prisma-Anordnung die folgenden Mantelflächenbereiche aufweist:
f) eine erste, insbesondere planare Eintrittsfläche für Referenzlicht, die sich entlang einer ersten Eintrittsebene erstreckt, wobei die erste Eintrittsfläche Teil einer ersten Stirnfläche sein kann und
g) eine zweite, insbesondere planare Eintrittsfläche für Objektlicht, die sich entlang einer zweiten Eintrittsebene erstreckt, wobei die erste und die zweite Eintrittsfläche gegenüberliegende Mantelflächenbereiche der zweiten Prisma-Anordnung ausbilden, wobei die zweite Eintrittsfläche Teil einer zweiten Stirnfläche sein kann,
h) eine Austrittsfläche, die sich entlang einer Austrittebene erstreckt und durch die gebeugtes Referenzlicht und gebeugtes Objektlicht aus dem zweiten Beugungselement austreten kann,
i) eine der Austrittsfläche gegenüberliegende Prismafläche, wobei eine optische Transmissions-Beugungsgitteranordnung im zweiten Beugungselement angeordnet ist, die sich entlang einer Beugungsebene, welche die erste Eintrittsebene und die Austrittsebene schneidet, zwischen der ersten Eintrittsfläche und insbesondere der Austrittsfläche des zweiten Beugungselements erstreckt, insbesondere so dass die Transmissions-Beugungsgitteranordnung mit der ersten Eintrittsfläche und der Austrittfläche des zweiten Beugungselements ein Dreiecksprisma bilden, insbesondere wobei die Beugungsebene einen imaginären Mantelflächenbereich des Dreiecksprismas bildet,
wobei die Transmissions-Beugungsgitteranordnung des zweiten Beugungselements zumindest ein erstes Volumen-Phasen-Hologramm Gitter umfasst, und dass die zweite Holografie-Anordnung auf Seite der Prismafläche einen zweiten insbesondere planaren Spiegel mit einer zweiten Spiegelebene aufweist, wobei die zweite Spiegelebene mit der Beugungsebene einen Winkel α' einschließt, und die Prismafläche einen Winkel ω'₂ mit der Beugungsebene einschließt, wobei mindestens einer oder beiden Winkel α', ω'₂ ungleich 45° betragen, insbesondere wobei der Winkel α' = 45° ± β' ist mit β' >0°, insbesondere wobei der Winkel ω'₂ = 45° ± ρ'₂ ist mit ρ'₂ >0°, insbesondere um einen maximal möglichen Wirkungsgrad für Objekt- und Referenzlicht in der zweiten Holografie-Anordnung zu erzielen.

Bevorzugt sind β' und/oder ρ'₂ größer als 0.2°

Bevorzugt sind β' und/oder ρ'₂ kleiner als 20°.

Gemäß dieser Ausführungsform der Erfindung sind die Winkel β' bzw. ρ'₂ insbesondere so gewählt, dass auf die Transmissions-Beugungsgitteranordnung unter einem Winkel von 45° auftreffendes Referenzlicht von der Transmissions-Beugungsgitteranordnung in Richtung Prismafläche des zweiten Beugungselements gebeugt wird und von dem zweiten Spiegel so reflektiert wird, dass das reflektierte Referenzlicht aufgrund eines Einfallswinkels des reflektierten Referenzlichtes auf die Transmissions-Beugungsgitteranordnung des zweiten Beugungselements zu einem geringeren Anteil, insbesondere einen um mindestens 30% geringeren Anteil, als das ursprünglich von Seite der ersten Eintrittsfläche des zweiten Beugungselements auf die Transmissions-Beugungsgitteranordnung treffendes Referenzlicht wieder in Richtung erster Eintrittsfläche des zweiten Beugungselements gebeugt wird und ein verbleibender Anteil des reflektierten Referenzlichtes durch die Transmissions-Beugungsgitteranordnung in Richtung Austrittsfläche des zweiten Beugungselements propagiert.

Im erfindungsgemäßen Zustand ist insbesondere vorgesehen, dass Referenzlicht über die erste Eintrittsfläche des zweiten Beugungselements, insbesondere kollimiert in das Beugungselement eintritt. Dort wird es durch die Transmissions-Beugungsgitteranordnung in Richtung Prismafläche des zweiten Beugungselements gebeugt. Durch den zweiten Spiegel wird das gebeugte Referenzlicht in Richtung der Transmissions-Beugungsgitteranordnung zurückreflektiert - allerdings unter einem Winkel, der sich aus dem Snell'schen Gesetz berechnen lässt, dadurch wird zumindest ein Teil des reflektierten Referenzlichtes von der Transmissions-Beugungsgitteranordnung nicht gebeugt, sondern transmittiert und propagiert in Richtung der Austrittsfläche des zweiten Beugungselements. Das Objektlicht tritt erfindungsgemäß über die zweite Eintrittsfläche in das zweite Beugungselement ein, und wird durch die Transmissions-Beugungsgitteranordnung direkt in Richtung Austrittsfläche gebeugt, wo es mit dem Referenzlicht an einem zweiten Array-Detektor zur Überlagerung und insbesondere Interferenz kommt.

Im erfindungsgemäßen Zustand des Systems ist insbesondere vorgesehen, dass Referenzlicht zunächst in erste Holografie-Anordnung eingestrahlt wird und Objektlicht zunächst in die zweite Holografie-Anordnung eingestrahlt wird.

Gemäß dieser Ausführungsform wird also eine zweite Holografie-Anordnung offenbart, die im Wesentlichen die gleichen Merkmale wie die erste Holografie-Anordnung aufweist, insbesondere wobei die Zentralwellenlängen der Transmissions-Beugungsgitteranordnung der ersten und zweiten Holografie-Anordnung unterschiedlich sind.

Gemäß einer weiteren Ausführungsform der Erfindung, weist das erstes Volumen-Phasen-Hologramm Gitter der zweiten Holografie-Anordnung eine von dem ersten und/oder dem zweiten Volumen-Phasen-Hologramm Gitter der ersten Holografie-Anordnung verschiedene Zentralwellenlänge auf.

Insbesondere sind die erste und die zweite Holografie-Anordnung so zueinander angeordnet, dass die Austrittflächen parallel und insbesondere ko-planar - also in der gleichen Ebene liegend - zueinander ausgerichtet sind, so dass beispielsweise möglich ist, einen einzigen Array-Detektor zum Aufzeichnen der Interferenzmuster an den Austrittsflächen beider Beugungselemente zu verwenden.

Gemäß einer alternativen Ausführungsform der Erfindung sind die erste und die zweite Holografie-Anordnung so zueinander angeordnet, dass die Austrittflächen des ersten und des zweiten Beugungselements parallel zueinander ausgerichtet sind, wobei die Austrittsfläche des ersten Beugungselements auf Seite der Prismafläche des zweiten Beugungselements angeordnet ist und die Austrittsfläche des zweiten Beugungselements aus Seite der Prismafläche des ersten Beugungselements angeordnet ist. Insbesondere ist die erste Holografie-Anordnung relativ zur zweiten Holografie-Anordnung insbesondere um die optische Achse um 180° gedreht. Insbesondere schließen die Beugungsebenen des ersten und des zweiten Beugungselements einen Winkel von zumeist 90° ein.

Dies bietet den Vorteil, dass zwei unabhängige Array-Detektoren verwendet werden können, und es weniger Bauraum-Problematiken bezüglich der Array-Detektoren gibt.

Gemäß einer weiteren Ausführungsform der Erfindung sind die erste und die zweite Holografie-Anordnung so zueinander angeordnet, dass die erste Eintrittsfläche des zweiten Beugungselements der zweiten Holografie-Anordnung gegenüber und insbesondere parallel zur der zweiten Eintrittsfläche des ersten Beugungselements der ersten Holografie-Anordnung angeordnet sind, insbesondere wobei die Beugungsebenen der ersten und der zweiten Beugungselemente optisch parallel zueinander ausgerichtet sind.

Hier sei noch einmal darauf hingewiesen, dass die Ausdrücke ,gegenüber' und ,parallel' im optisch funktionalen und nicht zwingend im streng geometrischen Sinne zu verstehen sind.

Gemäß einer Ausführungsform der Erfindung umfasst das zweite Beugungselement eine optische Achse, die sich zwischen der ersten und der zweiten Austrittfläche erstreckt und die Beugungsebene des zweiten Beugungselements in einem Winkel von 45° Winkel schneidet.

Insbesondere sind die erste und die zweite Holografie-Anordnung entlang einer gemeinsamen optischen Achse angeordnet und ausgerichtet, die der optischen Achse des ersten Beugungselements entspricht.

Es ist wird angemerkt, dass alle Merkmale, Vorteile und Ausführungsformen, die die erste Holografie-Anordnung, das erste Beugungselement, den ersten Spiegel oder andere Komponenten betreffen und in deren Zusammenhang offenbart sind in analoger Weise auf die möglichen Ausführungsformen der zweiten Holografie-Anordnung anwendbar sind und zur weiteren Detaillierung aus den vorhergehenden Absätzen entsprechend zur Spezifizierung der zweiten Holografie-Anordnung herangezogen werden können.

Der zweite Spiegel umfasst eine zweite Spiegelebene, die sich entlang einer reflektierenden Schicht des zweiten Spiegels erstreckt, insbesondere wobei der zweite Spiegel aus der reflektierenden Schicht besteht.

Im Folgenden ist unter einem Winkel des zweiten Spiegels bezüglich einer Fläche oder einer Ebene stets der Winkel der zweiten Spiegelebene des zweiten Spiegels mit der Fläche oder der entsprechenden Ebene gemeint.

Gemäß der Erfindung kann jeder Fläche, also der ersten und zweiten Eintrittsfläche, der Austrittsfläche und der Prismafläche des zweiten Beugungselements eine Ebene zugeordnet werden, die sich entlang der jeweiligen Fläche erstreckt. Wenn auf einen Winkel zwischen zwei Flächen Bezug genommen wird, so ist damit auch der Winkel zwischen den Ebenen gemeint. Die Winkelangabe beziehen sich sofern nicht explizit anders offenbart auf die innenliegenden Winkel der zweiten Prisma-Anordnung.

Gemäß einer Ausführungsform der Erfindung, schließt die Beugungsebene des zweiten Beugungselements eine Winkel ω'₁ mit der ersten Eintrittsfläche des zweiten Beugungselements ein, insbesondere wobei der Winkel 30° ≤ ω'₁ ≤ 60 °, insbesondere wobei ω'₁ = 45°.

Gemäß einer Ausführungsform der Erfindung, schließt die Beugungsebene des zweiten Beugungselements einen Winkel ω'₂ mit der Prismafläche des zweiten Beugungselements ein, insbesondere wobei der Winkel 30° ≤ ω'₂ ≤ 60 °, insbesondere wobei ω'₂ = 45° oder ω'₂ = 45° ± β', insbesondere wobei ω'₂ so gewählt ist, dass die Prismafläche mit der Spiegelebene des zweiten Spiegels parallel verläuft.

Gemäß einer Ausführungsform der Erfindung schließt die Beugungsebene des zweiten Beugungselements einen Winkel ω'₃ mit der zweiten Eintrittsfläche des zweiten Beugungselements ein, insbesondere wobei der Winkel 30° ≤ ω'₃ ≤ 60 °, insbesondere wobei ω'₃ = 45°.

Gemäß einer Ausführungsform der Erfindung, schließt die Beugungsebene des zweiten Beugungselements einen Winkel ω'₄ mit der Austrittsfläche des zweiten Beugungselements ein, insbesondere wobei der Winkel 30° ≤ ω'₄ ≤ 60 °, insbesondere wobei ω'₄ = 45° oder ω'₄ = 45° ± β', insbesondere wobei ω'₄ so gewählt ist, dass die Austrittsebene mit der Spiegelebene des zweiten Spiegels parallel verläuft.

Gemäß einer Ausführungsform der Erfindung sind die erste und die zweite Eintrittsfläche des zweiten Beugungselements parallel zueinander ausgerichtet.

Gemäß einer Ausführungsform der Erfindung sind die Prismafläche und die Austrittfläche des zweiten Beugungselements parallel zueinander ausgerichtet.

Gemäß einer weiteren Ausführungsform der Erfindung, betragen die Winkel ω'₁ und ω'₃ gleich 45°. In dieser Konfiguration ist es insbesondere möglich, Referenzlicht und Objektlicht senkrecht auf die erste und zweite Eintrittsfläche zu strahlen, da die Eintrittsflächen rechtwinklig auf die optische Achse des zweiten Beugungselements stehen.

Gemäß einer Ausführungsform der Erfindung sind die erste Eintrittsfläche des ersten Beugungselements und die zweite Eintrittsfläche des zweiten Beugungselements parallel zueinander ausgerichtet.

Gemäß einer Ausführungsform der Erfindung sind die Winkel β' und β unterschiedlich groß.

Die unterschiedlichen Winkel β' und β können vorteilhaft gemäß den Zentralwellenlängen und Bandbreiten der Volumen-Phasen-Hologramm Gitter in dem ersten und zweiten Beugungselement angepasst sein.

Gemäß einer alternativen Ausführungsform der Erfindung sind die Winkel β' und β identisch.

Gemäß einer weiteren Ausführungsform der Erfindung sind die Winkel ω'₁ und ω₁ identisch.

Gemäß einer weiteren Ausführungsform der Erfindung sind die Winkel ω'₂ und ω₂ identisch.

Gemäß einer weiteren Ausführungsform der Erfindung sind die Winkel ω'₃ und ω₃ identisch.

Gemäß einer weiteren Ausführungsform der Erfindung sind d die Winkel ω'₄ und ω₄ identisch.

Gemäß einer weiteren Ausführungsform der Erfindung weisen das erste und das zweite Beugungselement die gleiche Geometrie und insbesondere Größe auf, sind also bis auf die optische Charakteristik der Transmissions-Beugungsgitteranordnung identisch.

Die erste und/oder zweite Eintrittsfläche des ersten und/oder des zweiten Beugungselements können mit einer Antireflexionsbeschichtung versehen sein. Ebenso kann die Austrittsfläche, und/oder die Prismafläche des ersten und/oder des zweiten Beugungselements mit einer Antireflexionsbeschichtung versehen sein.

Im erfindungsgemäßen Zustand ist insbesondere vorgesehen, dass Referenzlicht über die erste Eintrittsfläche des ersten Beugungselements, insbesondere kollimiert in das erste Beugungselement eintritt. Dort wird es teilweise durch die Transmissions-Beugungsgitteranordnung in Richtung Prismafläche des ersten Beugungselements gebeugt. Ein weiterer Teil des Referenzlichtes, der aufgrund seiner Wellenlängen nicht an der Transmissions-Beugungsgitteranordnung des ersten Beugungselements gebeugt wird, propagiert in Richtung des zweiten Beugungselements und wird dort durch die Transmissions-Beugungsgitteranordnung des zweiten Beugungselements in Richtung Prismafläche des zweiten Beugungselements gebeugt.

Analoges gilt insbesondere auch für das Objektlicht, das zunächst über die zweite Eintrittsfläche des zweiten Beugungselements eintritt und dann teilweise weiter in Richtung des ersten Beugungselements propagiert. An den Austrittsflächen bzw. den Array-Detektoren des ersten und des zweiten Beugungselements kommt es dann dementsprechend spektral aufgelöst zur Überlagerung des Referenzlichtes mit dem Objektlicht.

Gemäß einer weiteren Ausführungsform der Erfindung, umfasst das System einen zweiten optischen Array-Detektor, wie beispielsweise eine Kamera, wobei der zweite Array-Detektor dazu eingerichtet ist, aus der Austrittfläche des zweiten Beugungselements austretendes Licht zu detektieren.

Insbesondere ist der zweite Array-Detektor auf Seite der Austrittfläche des zweiten Beugungselements der zweiten Holografie-Anordnung angeordnet.

Weiterhin kann vorgesehen sein, dass der zweite Array-Detektor auf der Austrittfläche des zweiten Beugungselements der zweite Holografie-Anordnung unter Vermeidung der Ausbildung eines Luftspalt, also direkt, angeordnet ist und insbesondere wobei der zweite Array Detektor mit der Austrittsfläche des zweite Beugungselements verbunden ist, so dass zwischen Austrittsfläche und Array-Detektor zumindest kein Luftspalt ist.

Weiterhin kann der zweite Array-Detektor dazu eingerichtet sein, das detektierte Licht in ein elektrisches Signal umzuwandeln, das von der Auswerteeinrichtung, beispielsweise einem Computer, des Systems zumindest hinsichtlich einer zweidimensionalen Intensitätsverteilung des detektierten Lichts auf dem zweite Array-Detektor ausgewertet werden kann.

Durch den zweiten Array-Detektor kann eine Detektionsebene gegeben sein, die sich entlang dem Array des zweiten Array-Detektors erstreckt, also beispielsweise entlang einer Pixelmatrix der Kamera.

Gemäß einer weiteren Ausführungsform der Erfindung ist vorgesehen, dass die Detektionsebene des zweiten Array-Detektors mit der Beugungsebene einen Winkel von ε' = 45° ± η' mit η' > 0° einschließt, insbesondere wobei η' = β', insbesondere wobei die Detektionsebene parallel zum zweiten Spiegel verläuft. Weiterhin kann die Austrittsebene parallel oder ko-planar zur Detektionsebene verlaufen.

Insbesondere ist 0°< η' < 20°.

Gemäß einer Ausführungsform der Erfindung kann vorgesehen sein, dass die Detektionsebene eine solchen Winkel mit der Beugungsebene des zweiten Beugungselements einschließt, dass von der Detektionsebene reflektiertes Licht unter einer Littrow-Geometrie auf die Beugungsebene trifft, so dass das reflektierte Licht von der Transmissions-Beugungsgitteranordnung der zweiten Holografie-Anordnung vollständig oder zumindest größtenteils in Richtung der zweiten Eintrittsfläche gebeugt des zweiten Beugungselements wird und dort austreten kann. Somit kann eine durch mehrfach reflektiertes Licht entstehendes "ghosting"-Signal auf dem zweiten Array-Detektor vermieden werden.

Die vorerwähnte Ausbildungsform der Erfindung hat den Vorteil, dass verhindert werden kann, dass vom zweiten Array-Detektor rückreflektiertes Licht abermals zum zweiten Spiegel gelangt und von dort wiederum auf den zweiten Array-Detektor reflektiert wird und dort ein ungewolltes Signal erzeugt. Durch Wahl des Winkels η', kann das vom zweiten Array-Detektor reflektierte Licht vollständig oder größtenteils von der ersten Transmissions-Beugungsgitteranordnung in Richtung der zweiten Eintrittsfläche des zweiten Beugungselements gebeugt werden.

Gemäß einer weiteren Ausführungsform der Erfindung ist vorgesehen, dass die Grundfläche der zweiten Prisma-Anordnung ein Parallelogramm bildet, insbesondere wobei die Beugungsebene des zweiten Beugungselements entlang einer Diagonalen verläuft, die sich in der Grundfläche zwischen der ersten Eintrittsfläche und der Austrittsfläche des zweiten Beugungselements erstreckt, insbesondere wobei
i) die erste Eintrittsfläche mit der Beugungsebene des zweiten Beugungselements einen Winkel ω'₁ = 45°± ρ'₁ einschließt und die Prismafläche mit der Beugungsebene des zweiten Beugungselements einen Winkel ω'₂ = 45° einschließt, wobei ρ'₁ > 0°, insbesondere wobei ρ'₁ > 0.2°, oder
ii) die erste Eintrittsfläche mit der Beugungsebene des zweiten Beugungselements einen Winkel ω'₁ = 45° einschließt und die Prismafläche mit der Beugungsebene des zweiten Beugungselements einen Winkel ω'₂ = 45°± ρ'₂ einschließt, wobei ρ'₂ > 0°, insbesondere wobei ρ'₂ > 0.2°, oder
iii) die erste Eintrittsfläche mit der Beugungsebene des zweiten Beugungselements einen Winkel ω'₁ = 45°± ρ'₁ einschließt und die Prismafläche mit der Beugungsebene des zweiten Beugungselements einen Winkel ω'₂ = 45°± ρ'₂ einschließt, wobei ρ'₁ > 0° und ρ'₂ > 0°, insbesondere wobei ρ'₁ > 0.2° und ρ'₂ > 0.2°, oder
iv) die erste Eintrittsfläche mit der Beugungsebene des zweiten Beugungselements einen Winkel ω'₁ = 45° einschließt und die Prismafläche mit der Beugungsebene des zweiten Beugungselements einen Winkel ω'₂ = 45°einschließt, die Grundfläche ein spezielles Parallelogramm ist, nämlich ein Rechteck, vorzugsweise ein Quadrat, insbesondere wobei die Beugungsebene des zweiten Beugungselements entlang einer Diagonalen verläuft, die sich in der Grundfläche zwischen der ersten Eintrittsfläche und der Austrittsfläche des zweiten Beugungselements erstreckt.

Jede der Ausführungsformen i) bis iii) reduziert potenzielle Störreflexe, die beim Ein- oder Austritt von Referenz und/oder Objektlicht in die zweite Prisma-Anordnung entstehen, in vorteilhafter Weise.

Besonders die Ausführungsformen ii) und iii) ermöglichen, dass der zweite Spiegel direkt auf der Prismafläche des zweiten Beugungselements angeordnet ist, wenn beispielsweise der Winkel ρ'₂ = β' gewählt ist.

Weiterhin kann in den Ausführungsformen ii) und iii) der zweite Array-Detektor ebenfalls direkt auf der Austrittfläche angeordnet sein, wie oben bereits erwähnt.

Dabei kommen die Vorteile einer reflexarmen Geometrie und die zuvor zum Array-Detektor aufgeführten Vorteile bezgl. einer Rückreflexion vom Array Detektor in Littrow-Konfiguration besonders zum Tragen, da der Winkel zwischen Detektionsebene und Beugungsebene ε' = 45° ± η' mit η' = β' = ρ'₂ beträgt, so dass die Detektionsebene parallel zum zweiten Spiegel verläuft. Weiterhin verläuft die Austrittsebene im Wesentlichen ko-planar zur Detektionsebene.

Insbesondere, wenn zumindest die zweite Eintrittsfläche entspiegelt ist, ist der an der zweiten Eintrittsfläche wieder zurückreflektierte Anteil des Referenzlichtes (der vom zweiten Array-Detektor reflektiert und über die Transmissions-Beugungsgitteranordnung gebeugt wurde zu vernachlässigen. Je nach Strahl-Konfiguration kann es aber auch einen Schrägstellungswinkel an der Austrittsfläche geben (z.B. Ausführungsform iii), so dass eine zusätzliche Rückreflexunterdrückung erreicht wird.

Die Ausführungsformen i) und iii) erlauben in vorteilhafter Weise gleichzeitig eine Polarisationsselektion unter Ausnutzung des Brewsterwinkels und ggf. polarisationsdrehenden Elementen, wie einer λ/2 Platte, durchzuführen und/ oder durchzuführen und/ oder eine Reduktion von Reflexionen zu erreichen. Referenzlicht und Objektlicht sollten dann unter einem Winkel (also ungleich 90°) so in das erste Beugungselement gekoppelt werden, dass das Referenzlicht und das Objektlicht um die Zentralwellenlänge in einem Winkel von 45° auf die Transmissions-Beugungsgitteranordnung auftreffen, also in Littrow-Konfiguration.

Gemäß der Ausführungsform iv), ist die Grundfläche der zweiten Prisma-Anordnung rechteckig, insbesondere quadratisch ausgebildet.

Die Ausführungsform iv) stellt ein einfach herzustellendes zweites Beugungselement zur Verfügung. In dieser Konfiguration kann sowohl das Referenzlicht als auch das Objektlicht senkrecht zur jeweiligen Eintrittsfläche eingestrahlt werden und trifft dadurch in der Littrow-Konfiguration auf die Transmissions-Beugungsgitteranordnung. Weiterhin ist ein einfacher Weise der Winkel α' des zweiten Spiegels und ggf. der Winkel ε' des zweiten Array-Detektors einstellbar.

Gemäß einer weiteren Ausführungsform der Erfindung ist der zweite Spiegel auf der Prismafläche der zweiten Holografie-Anordnung angeordnet.

Diese Ausführungsform der Erfindung umfasst die Möglichkeit, dass der zweite Spiegel als reflektierende Schicht auf der Prismafläche ausgebildet ist. Die reflektierende Schicht kann durch bekannte Verfahren auf der Prismafläche erzeugt werden.

Weiterhin erlaubt diese Ausführungsform ist eine besonders robuste und kompakte Ausführungsform der Erfindung, da der zweite Spiegel direkt mit dem zweiten Beugungselement verbunden ist und somit keine Montage oder Justage des Spiegels notwendig ist. Impliziter Weise schließt die Prismafläche dann den Winkel ω'₂ = α' mit der Beugungsebene des zweiten Beugungselements ein.

Gemäß einer weiteren Ausführungsform der Erfindung, ist der Winkel β' über den zweiten Spiegel einstellbar oder eingestellt.

Gemäß einer weiteren Ausführungsform der Erfindung, ist der zweite Spiegel um zumindest eine Achse kippbar, so dass der Winkel β' über den zweiten Spiegel einstellbar oder eingestellt ist.

Im Zusammenhang mit dieser Ausführungsform ist der zweite Spiegel insbesondere als separates Element ausgeführt, das optional so ausgebildet ist, dass der Winkel β' einstellbar ist.

Gemäß einer weiteren Ausführungsform der Erfindung, umfasst die optische Transmissions-Beugungsgitteranordnung des zweiten Beugungselements ein zweites Volumen-Phasen-Hologramm Gitter, das sich insbesondere ebenfalls entlang bzw. auf der Beugungsebene erstreckt, wobei das erste und das zweite Volumen-Phasen-Hologramm Gitter des zweiten Beugungselements eine maximale Beugungsintensität, insbesondere für die erste Beugungsordnung, bei verschiedene Zentralwellenlängen aufweisen, insbesondere wobei Zentralwellenlängen einen Abstand von mindestens der Bandbreite des ersten und des zweiten Volumen-Phasen-Hologramm Gitters des zweiten Beugungselements aufweisen. So kann insbesondere gewährt werden, dass eine spektrale Auftrennung der Zentralwellenlängen an der Austrittsfläche des zweiten Beugungselements gewährleistet ist.

Diese Ausführungsform erlaubt es im Vergleich zu einem einzigen Volumen-Phasen-Hologramm Gitter einen vergrößerten Spektralbereich für das System nutzbar zu machen, d.h. es können mehrere verschiedene Wellenlängen insbesondere spektrale Linien über das zweite Beugungselement spektral an der Austrittsfläche aufgetrennt werden, ohne dass das zweite Beugungselement mehr Bauraum benötigt.

Gemäß einer Ausführungsform der Erfindung weist das erste Volumen-Phasen-Hologramm Gitter des zweiten Beugungselements beispielsweise eine Zentralwellenlänge von 940 nm und eine Bandbreite von 10 nm auf.

Gemäß einer Ausführungsform der Erfindung weist das zweite Volumen-Phasen-Hologramm Gitter des zweiten Beugungselements beispielsweise eine Zentralwellenlänge von 960 nm und eine Bandbreite von 10 nm auf.

Gemäß dieser beiden Ausführungsformen überlappen die Wellenlängenbereiche des ersten und des zweiten Volumen-Phasen-Hologramm Gitters nicht, da die Zentralwellenlängen 20 nm voneinander getrennt sind, was der Summe der Bandbreiten des ersten und des zweiten Volumen-Phasen-Hologramm Gitters des zweiten Beugungselements entspricht. Je größer der Abstand der Zentralwellenlängen innerhalb der Bandbreiten ist, desto weniger Wechselwirkungen sind zu erwarten.

Gemäß einer weiteren Ausführungsform der Erfindung umfasst die Transmissions-Beugungsgitteranordnung des zweiten Beugungselements mehr als zwei Volumen-Phasen-Hologramm Gitter, insbesondere wobei die Volumen-Phasen-Hologramm Gitter unterschiedliche Zentralwellenlängen haben. Die Zentralwellenlängen können nach dem oben ausgeführten Prinzip gemäß der Bandbreiten oder mehr spektral beabstandet sein.

In Kombination mit der ersten Holografie-Anordnung kann so eine zweiter Spektralbereich zur digitalen Holografie verwendet werden.

Insbesondere sind die Transmissions-Beugungsgitteranordnungen des ersten und des zweiten Beugungselements für verschiedene Zentralwellenlängen konfiguriert, d.h. auch hier sind die Zentralwellenlängen der Transmissions-Beugungsgitteranordnungen nach dem oben ausgeführten Prinzip gemäß der Bandbreiten oder mehr spektral beabstandet sein.

Gemäß einer weiteren Ausführungsform der Erfindung ist vorgesehen, dass die zweite Eintrittsfläche des ersten Beugungselements mit der ersten Eintrittsfläche des zweiten Beugungselements verbunden ist, insbesondere verklebt oder verschweißt ist, insbesondere wobei das erste Beugungselement entlang seiner zweiten Eintrittsfläche integral mit der ersten Eintrittsfläche des zweiten Beugungselements ausgeformt ist.

Die Ausführungsform erlaubt einen kompakten justagefreien Aufbau des optischen Systems, da zumindest das erste und das zweite Beugungselement eine starre Ausrichtung und Position zueinander haben. Sofern der erste und der zweite Spiegel ebenfalls integral mit dem Beugungselement ausgebildet sind, erhöht sich die Robustheit. Weiterhin kann sowohl das erste als auch der zweite Array-Detektor mit dem jeweiligen Beugungselement verbunden sein.

Ja nach Art der Verbindung der jeweiligen Eintrittsflächen kann es sein, dass die verbundenen Eintrittsflächen eine im Wesentlichen imaginäre Natur besitzen (z.B. da sie miteinander verschmolzen sind). Insbesondere jedoch soll im Sinne der Erfindung selbst eine monolithische Ausführung des ersten und zweiten Beugungselements zumindest im Rahmen der Auslegung und dem allgemeinen Verständnis des Fachmanns umfasst sein.

Gemäß einer weiteren Ausführungsform der Erfindung weist das optische System auf Seite der ersten Eintrittsfläche der ersten Holografie-Anordnung eine Kollimationsoptik für Referenzlicht auf, die eine optische Achse aufweist, welche in Richtung der ersten Eintrittsfläche der ersten Holografie-Anordnung verläuft, insbesondere wobei die optische Achse lateral, also versetzbar verläuft, und wobei die Kollimationsoptik dazu eingerichtet ist, Laserlicht in Form von Referenzlicht vor Eintritt durch die erste Eintrittsfläche des ersten Beugungselements zu kollimieren. Die Kollimationsoptik ist insbesondere so mit ihrer optischen Achse ausgerichtet, dass von ihr kollimiertes Referenzlicht nach Eintritt in das erste Beugungselement entlang der optischen Achse des ersten Beugungselements propagiert, so dass es in einem 45° Winkel auf die Beugungsebene des ersten Beugungselements trifft. Der laterale Versatz kann so ausgestaltet sein, dass das kollimierte Licht nach Reflexion an dem Referenzspiegel mit seinem Winkel β relativ zur optischen Achse wieder zentrisch auf die Austrittsfläche bzw. das Detektor-Array trifft.

Der Begriff "kollimiert" ist dabei insbesondere so auszulegen, dass das Referenzlicht zumindest für eine Wellenlänge, nur minimale Wellenfrontenkrümmung aufweist. Bei abweichenden Wellenlängen erhöht sich die Konvergenzen oder Divergenzen des Lichtstrahles bzw. Wellenfeldes je nach Wellenlänge. Auch diese chromatisch bedingten Abweichungen von der idealen Kollimierung sind mit dem Begriff "kollimiert" im Kontext dieser Erfindung umfasst. Zudem sind Justage- und System-Toleranz-bedingte Abweichungen ebenso unter dem Begriff "kollimiert" zu verstehen. Insbesondere ist unter dem Begriff "kollimiert" auch eine Konvergenz und/oder Divergenz des Lichtstrahls bis zu einem Divergenz- oder Konvergenzwinkel ξ der im Bereich 0°< ξ ≤ 2*β oder 2*ρ₂ und/oder 0°< ξ ≤ 2*β' oder 2*ρ'₂ liegt, im Rahmen der Erfindung zu verstehen.

Gemäß einer weiteren Ausführungsform der Erfindung, umfasst das optische System eine Objektivlinse für Objektlicht, die eine optische Achse aufweist, welche in Richtung der zweiten Eintrittsfläche der ersten und/oder der zweiten Holografie-Anordnung verläuft, wobei die Objektivlinse so im optischen System angeordnet ist, dass von der Objektivlinse in Richtung zweiter Eintrittsfläche des ersten und/oder des zweiten Beugungselements propagierendes Licht kollimiert ist, wenn das Licht in und/oder nahe einer Brennebene der Objektivlinse in Richtung der Objektivlinse abgestrahlt, insbesondere reflektiert oder gestreut wird, insbesondere wobei das Licht Objektlicht ist.

Der Begriff "nahe" in Bezug auf ein eine Brennebene oder einen Fokus- oder Brennpunkt im Kontext der Spezifikation ist insbesondere als Toleranzangabe zu verstehen, da Abweichungen aus der idealen optischen Anordnung der Komponenten bzw. Abweichungen von einem idealen Strahlen-/Wellenverlauf des Lichtes vom erfindungsgemäßen System ohne weiteres gehandhabt werden können. In diesem Zusammenhang kann der Begriff "nahe" beispielsweise als Toleranzbereich in Bezug auf eine Brennweite der Objektivlinse verstanden werden, so dass bei einer nominalen Brennweite *f*, ein Abweichungsbereich von ± *f*/5 durch den Begriff "nahe" definiert wird. Ein analoges Verständnis kann auch für die weiteren Komponenten des Systems gelten.

Gemäß der Gesetzte der Abbildungsoptik, ist das Licht welches von der Brennebene der Objektivlinse näher bzw. weiter entfernt ist divergent bzw. konvergent, wenn es auf die zweite Eintrittsfläche trifft. Je nach Grad der Divergenz/Konvergenz kann aber auch dieses Licht zur Bildgebung im digitalen Holografie-Verfahren beitragen.

Die Objektivlinse ist mit ihrer optischen Achse insbesondere so ausgerichtet, dass aus einem Brennpunkt der Objektivlinse stammendes Objektlicht nach Eintritt in das erste bzw. in das zweite Beugungselement entlang der optischen Achse des ersten bzw. des zweiten Beugungselements propagiert, so dass es in einem 45° Winkel auf die Beugungsebene des ersten bzw. des zweiten Beugungselements trifft.

Die Objektivlinse kann aus einer oder mehreren Linsen oder Linsenelementen bestehen.

Es ist weiterhin anzumerken, dass erfindungsgemäß insbesondere nur eine Objektivlinse vorgesehen ist, auch wenn zwei Holografie-Anordnungen im System umfasst sind. Die Objektivlinse ist dann näher auf Seite des zweiten Beugungselements und dessen zweiter Eintrittsfläche verortet.

Gemäß einer weiteren Ausführungsform der Erfindung umfasst das System die folgenden Komponenten:
- eine Laserlichtquelle, die dazu eingerichtet ist, Laserlicht mit einer oder mehreren Zentralwellenlängen zu Verfügung zu stellen, insbesondere wobei die Laserlichtquelle einen oder mehrere Laser umfasst, insbesondere wobei jeder Laser dazu eingerichtet ist, Laserlicht umfassend mindestens eine Zentralwellenlänge zu emittieren, wobei jeder Laser in einem Wellenlängenbereich um die Zentralwellenlänge des Lasers mindestens eine, vorzugsweise eine Vielzahl an Laserlinien emittiert,
- eine erste optische Faser, insbesondere eine polarisationserhaltende single-mode Faser, die dazu eingerichtet ist, das Laserlicht der Laserlichtquelle zu einer Eingangsapertur der Kollimationsoptik zu leiten, so dass die Kollimationsoptik das Laserlicht kollimiert in Form von Referenzlicht zur ersten Holografie-Anordnung leiten kann,
- eine zweite optische Faser, insbesondere eine polarisationserhaltende single-mode Faser, die dazu eingerichtet ist, das Laserlicht der Laserlichtquelle zu einer Ausgangsapertur des optischen Systems zu leiten, von der aus ein zu erfassendes Objekt mit Laserlicht in Form von Objektlicht zu beleuchtet wird.

Diese Ausführungsform erlaubt es, die Polarisation des Referenz- und des Objektlichtes zu kontrollieren. Insbesondere beugt ein Volumen-Phasen-Hologramm Gitter s-polarisiertes Licht signifikant besser p-polarisiertes Licht, so dass das System insbesondere dazu eingerichtet ist, dass das Referenzlicht und Objektlicht insbesondere s-polarisiert ist, nachdem es aus der ersten und zweiten optischen Faser austritt.

Gemäß einer weiteren Ausführungsform der Erfindung umfasst das System einen Fasersplitter, insbesondere polarisationserhaltenden Fasersplitter, der dazu ausgebildet ist, das Laserlicht der Laserlichtquelle zu teilen und in die mindestens eine erste und zweite optischen Faser einzukoppeln, insbesondere wobei der Fasersplitter und die mindestens eine erste und zweite optische Faser in einem integriert optischen Element umfasst sind.

Gemäß einer Ausführungsform der Erfindung, weist das System eine Abbildungsoptik auf, die dazu eingerichtet ist, Objektlicht, insbesondere aus der zweiten Faser austretendes Objektlicht, in Form eines Intensitätsmusters insbesondere auf ein zu erfassendes Objekts zu projizieren, wobei das Intensitätsmuster aus mindestens einem beleuchteten Bereich vorzugsweise aber einer Vielzahl an disjunkten beleuchteten Bereichen besteht, insbesondere wobei die beleuchteten Bereiche des Musters punkt- oder kreisförmig sind, insbesondere wobei das vom Objekt zurückgeworfene Objektlicht durch die Objektivlinse erfasst wird.

Diese Form des Intensitätsmusters erlaubt, eine parallele Aufnahme und Auswertung mehrere Objektbereiche mit einer Vielzahl an Wellenlängen.

Eine vollständige Erfassung kann durch eine Verlagerung des Musters auf dem Objekt erfolgen, so dass das Objekt bereichsweise optisch abgetastet und erfasst wird.

Auf diese Weise kann eine Oberfläche eines Objekts mit dem Intensitätsmuster abgetastet werden, wobei zu jedem Bereich eine Tiefeninformation sowie eine laterale Ortsinforation über erste und/oder zweite Holografie-Anordnung erzeugt werden kann, so dass eine 3D-Information von der Objektoberfläche, die mit dem Objektlicht abgetastet wird, erzeugt werden kann.

Gemäß einer weiteren Ausführungsform der Erfindung weist das System ein insbesondere wasserdichtes Gehäuse auf, wobei das Gehäuse die erste und/oder die zweite Holografie-Anordnung, die erste Kollimationsoptik und die Objektivlinse umfasst, insbesondere wobei die Laserlichtquelle außerhalb des Gehäuses angeordnet ist und über zumindest die flexible optische Faserleitung mit dem Gehäuse verbunden ist.

Insbesondere kann die Laserlichtquelle entweder im Gehäuse oder außerhalb davon angeordnet sein, und eingerichtet ist, aus der Laserlichtquelle stammendes Referenzlicht und dazu kohärentes Objektlicht über einen Fasersplitter vorzugsweise im Gehäuse in die mindestens eine erste und zweite Faser zu koppeln.

Ein solches optisches System mit Laserlichtquelle und Fasersplitter kann in einem vergleichsweise kompakten Modul integriert werden, wobei zumindest ein Referenzarm des Systems, also der Teil des Systems durch den Referenzlicht geleitet wird, und insbesondere die erste und oder zweite Holografie-Anordnung, Teil des integrierten Moduls sein kann. Ein solches System benötigt nur minimalen Bauraum innerhalb des Gehäuses, so dass es für minimal-invasive medizinische Applikationen geeignet ist.

Gemäß einer weiteren Ausführungsform der Erfindung ist das System dazu eingerichtet, das Laserlicht zur Verfügung zu stellen, welches Wellenlängen aus zumindest zwei Wellenlängenbereichen umfasst, wobei ein erster Wellenlängenbereich der zwei Wellenlängenbereiche um eine Zentralwellenlänge gelagert ist und Wellenlängen insbesondere in Form von spektralen Linien, z.B. in Form von Laserlinien umfasst, die außerhalb eines zweiten Wellenlängenbereichs liegen, wobei der zweite Wellenlängenbereich um eine andere Zentralwellenlänge gelagert ist der zwei Wellenlängenbereiche und Wellenlängen insbesondere in Form spektralen Linien umfasst, wobei insbesondere der erste und der zweite Wellenlängenbereich jeweils einen spektralen Bereich von nicht mehr als 50 nm umfassen, insbesondere nicht mehr als 15 nm, insbesondere wobei die spektralen Linien der Wellenlängenbereiche jeweils eine Linienbreite von nicht mehr als 0,5 nm insbesondere nicht mehr als 0.2 nm aufweisen.

Insbesondere weist ein solches System dann ein zwei Volumen-Phasen-Hologramm Gitter auf, die jeweils für eine der beiden Zentralwellenlängen konfiguriert sind, also dazu eingerichtet sind, die Zentralwellenlänge für die das Volumen-Phasen-Hologramm Gitter eingerichtet ist, zu beugen und die andere Zentralwellenlänge ungebeugt zu lassen.

Die beiden Volumen-Phasen-Hologramm Gitter können beide im ersten Beugungselement oder im zweiten Beugungselement angeordnet sein. Alternativ, kann jedes der beiden Beugungselemente jeweils eines dieser Volumen-Phasen-Hologramm Gitter umfassen.

Ein weiterer Aspekt der Erfindung betrifft ein human- oder veterinär-medizinisches Endoskop-System, das das optische System gemäß der Erfindung umfasst, um digital-holografische Aufnahmen zu erstellen.

Das Endoskop-System kann dazu eingerichtet sein, holografische Video-Aufnahmen zu erstellen und eine 3D-Repräsentation des erfassten Objekts auf einem Bildschirm anzuzeigen.

### Figurenbeschreibung

Weitere Merkmale und Vorteile der Erfindung werden nachfolgend anhand der Figurenbeschreibung von Ausführungsbeispielen erläutert. Winkel und Längen können in den Figuren übertrieben oder untertrieben dargestellt sein und dienen lediglich der Illustration der Erfindung. Insbesondere ist es vorteilhaft, wenn jede der Eintrittsflächen des Beugungselements größer als dessen Austrittsfläche ist.

Es zeigen:
- Fig. 1: eine schematische Schnittansicht einer ersten allgemeinen Ausführungsform der Holografie-Anordnung des erfindungsgemäßen Systems;
- Fig. 2: eine schematische Schnittansicht einer zweiten Ausführungsform der Holografie-Anordnung des erfindungsgemäßen Systems;
- Fig. 3: eine schematische Schnittansicht einer dritten Ausführungsform der Holografie-Anordnung des erfindungsgemäßen Systems;
- Fig. 4: eine schematische Schnittansicht einer vierten Ausführungsform der Holografie-Anordnung des erfindungsgemäßen Systems;
- Fig. 5: eine schematische Schnittansicht einer Ausführungsform des erfindungsgemäßen Systems mit einer Holografie-Anordnung;
- Fig. 6: eine schematische Schnittansicht einer Ausführungsform des erfindungsgemäßen Systems mit zwei Holografie-Anordnungen;

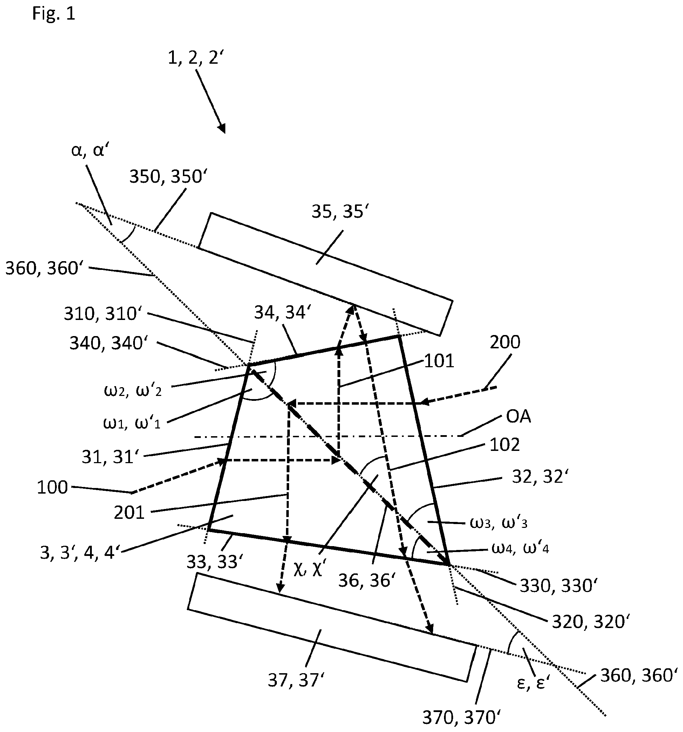

In Fig. 1 ist eine beispielhafte allgemeine Ausführungsform des Systems 1 gemäß der Erfindung schematisch als 2D-Schnitt dargestellt.

Das optische System 1 weist eine erste Holografie-Anordnung 2 auf, die ein erstes Beugungselement 3, einen ersten Spiegel 35 sowie einen ersten Array-Detektor 37 umfasst.

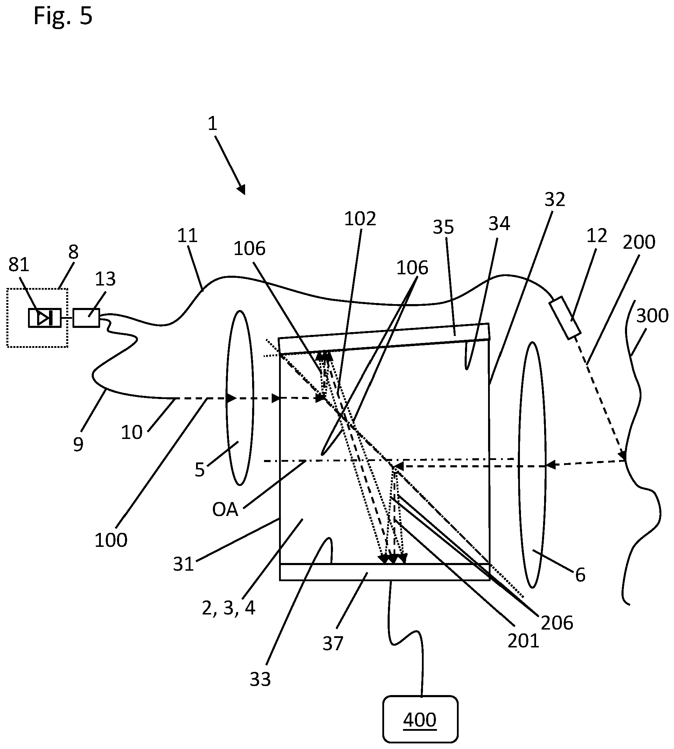

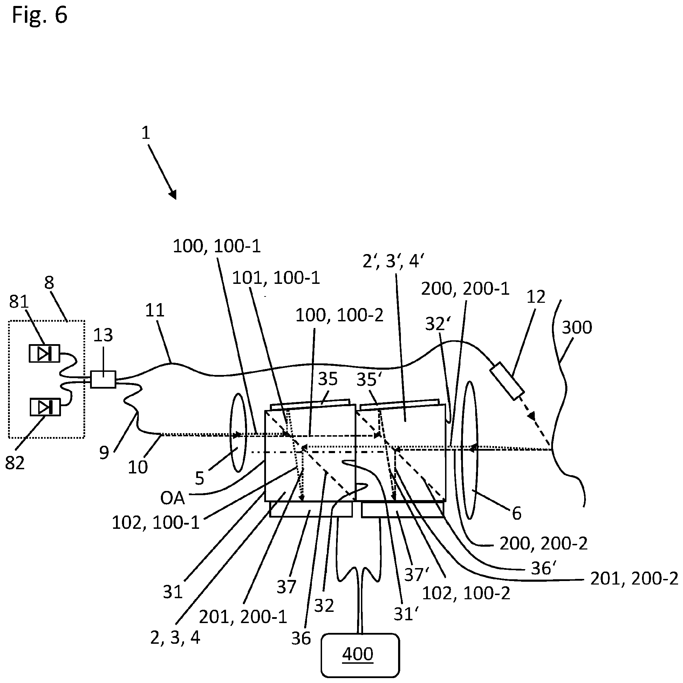

Das System 1 weist weiterhin eine Kollimationsoptik 5 und eine Objektivlinse 6 (vgl. Fig. 5 und 6) auf. Eine Laserlichtquelle 8 des Systems 1 ist dazu eingerichtet (vgl. Fig. 5 und 6), Referenzlicht 100 und Objektlicht 200 für das System 1 bereitzustellen.

Eine Variante des Systems kann eine zweite Holografie-Anordnung 2' umfassen, die ihrerseits ein zweite Beugungselement 3', einen zweiten Spiegel 35 sowie einen zweiten Array-Detektor 37 umfasst. Da wesentliche Merkmale der erste und zweiten Holografie-Anordnung 2, 2' in gleicher Weise angeordnet sind und funktional in gleicher Weise zusammenhängen, sind in den Figuren 1 bis 4 die entsprechenden Bezugszeichen der Elemente und Kompenenten auch für die zweiten Holografie-Anordnung 2' angegeben. Die Bezugszeichen für die zweite Holografie-Anordnung 2' entsprechen den Bezugszeichen zur ersten Holografie-Anordnung 2 zuzügliche eines " ' ". Im folgenden Text wird zunächst die erste Holografie-Anordnung 2 beschrieben, wobei gleiches oder zumindest analoges auch für die zweite Holografie-Anordnung 2' gilt.

Die erste Holografie-Anordnung 2 umfasst eine massive (z.B. Glas oder Polymer) Prisma-Anordnung 4, welche das erste Beugungselement 3 bildet, mit in diesem Fall viereckiger Grundfläche, deren Kanten von der Mantelfläche der Prisma-Anordnung 4 umfasst werden. Die Mantelfläche umfasst für vier Mantelflächenbereiche, nämlich eine erste und eine zweite Eintrittsfläche 31, 32, die jeweils Teil einer Stirnfläche des ersten Beugungselements 3 sein können, eine Austrittsfläche 33 sowie eine Prismafläche 34. Bis ggf. auf die Prismafläche 34, die auch reflektierend ausgestaltet sein kann, sind die erste und zweite Eintrittsfläche 31, 32 und die Austrittfläche 33 transparent.

Jeder Fläche der Prisma-Anordnung 4 kann eine entsprechende Ebene (jeweils als gepunktete Linien eingezeichnet) zugeordnet werden, die sich entlang der jeweiligen Fläche erstreckt. Entlang der ersten Eintrittsfläche 31 erstreckt sich demnach die erste Eintrittsebene 310, entlang der zweiten Eintrittsfläche 32 erstreckt sich die zweite Eintrittsebene 320, entlang der Austrittfläche 33 erstreckt sich die Austrittsebene 330 und entlang der Prismafläche 34 erstreckt sich die Prismaebene 340.

Die erste Prisma-Anordnung 4 umfasst weiterhin eine Transmissions-Beugungsgitteranordnung 36, die sich entlang einer Beugungsebene 360 im ersten Beugungselement 3 erstreckt. Die Transmissions-Beugungsgitteranordnung 36 umfasst zumindest ein erstes Volumen-Phasen-Hologramm Gitter.

Durch den ersten Spiegel 35 erstreckt sich entlang einer reflektierenden Fläche des ersten Spiegels 35 eine erste Spiegelebene 350. Der erste Spiegel 35 ist auf Seite der Prismafläche 34 angeordnet.

Zwischen der ersten und der zweiten Eintrittsfläche 31, 32 kann eine optische Achse OA des ersten Beugungselements 3 definiert werden, welche die Beugungsebene 360 in einem 45° Winkel schneidet.

Die erste Holografie-Anordnung 2 ist nun so konfiguriert, dass die erste Spiegelebene 350 mit der Beugungsebene 360 des ersten Beugungselements 3 einen Winkel α einschließt und wobei die Prismaebene 340 einen Winkel ω₂ mit der Beugungsebene 360 einschließt , wobei entweder beide, oder nur einer dieser Winkel α , ω₂ so gewählt ist, dass von der Transmissions-Beugungsgitteranordnung 36 in Richtung Prismafläche 34 gebeugtes Referenzlicht 101 von dem ersten Spiegel 35 so reflektiert wird, dass das reflektierte Referenzlicht 102 aufgrund eines Einfallswinkels des reflektierten Referenzlichtes 102 auf die Transmissions-Beugungsgitteranordnung 36, insbesondere zu einem geringeren Anteil, insbesondere weniger als 30%,als das ursprünglich auf die Transmissions-Beugungsgitteranordnung treffendes Referenzlicht 100 wieder in Richtung der ersten Eintrittsfläche 31 gebeugt wird und der verbleibende Anteil des reflektierten Referenzlichtes 102 durch die Transmissions-Beugungsgitteranordnung 36 in Richtung Austrittsfläche 33 propagiert.

Dieses Ziel wird ausgehend von einer Littrow-Konfiguration, also wenn in das Beugungselement 3 eingestrahltes und kollimiertes Referenzlicht 100 entlang der optischen Achse OA des ersten Beugungselements 3 propagiert, besonders gut erreicht, wenn der Winkel α = 45° ± β, mit β≠ 0° beträgt und/oder wenn der Winkel ω₂ = 45° ± ρ₂, mit ρ₂ ≠ 0° liegt bzw. liegen. Insbesondere sollte der Winkel β und/oder der Winkel ρ₂ betragsmäßig im Bereich 0.2° und 20° insbesondere im Bereich von 0.5° und 20° liegen, so dass α im Bereich 25° bis 44,5°bzw 45,5° bis 65° liegt und/oder so dass ω₂ im Bereich 25° bis 44,5°bzw 45,5° bis 65° liegt.

Zum Erreichen des angestrebten Effekts reicht es, wenn entweder der erste Spiegel 35 oder die Prismafläche 34 einen Winkel α oder ω₂ ungleich 45° einnehmen. Es ist aber, wie in Fig. 1 dargestellt, möglich, dass sowohl der erste Spiegel 35 als auch die Prismafläche 34 einen Winkel ungleich 45° mit der Beugungsebene einschließen.

Erfindungsgemäß ist insbesondere vorgesehen, dass Referenzlicht 100 in kollimiertem Zustand in einem Winkel von 45° auf die Transmissions-Beugungsgitteranordnung 36 trifft. Kollimierung und Propagationsrichtung des Referenzlichtes im Beugungselement 3 kann über die Position und Orientierung der Kollimationsoptik 5 (nicht in Fig. 1 dargestellt) erreicht werden.

Gemäß der Erfindung kann das Referenzlicht 100 zumindest eine Zentralwellenlänge 100-1 umfassen, bei der die Transmissions-Beugungsgitteranordnung 36 die erste Beugungsordnung um 90° relativ zur optischen Achse OA des Beugungselements 3 in der Littrow-Konfiguration beugt, so dass das gebeugte Referenzlicht 102 dieser Wellenlänge 100-1 um 45° von der Transmissions-Beugungsgitteranordnung 36 wegpropagiert. Abhängig von dem Winkel ω₂ der Prismafläche 34 und dem Winkel α des ersten Spiegels 35 wird erreicht, dass das gebeugte und von dem Spiegel 35 reflektierte Referenzlicht 102 der Zentralwellenlänge 100-1 nicht mehr im 45° Winkel auf die Transmissions-Beugungsgitteranordnung trifft. Sofern der Winkel χ, unter dem das reflektierte Referenzlicht 102 auf die Transmissions-Beugungsgitteranordnung 36 trifft, außerhalb der Bandbreite der Transmissions-Beugungsgitteranordnung 36 liegt, propagiert das reflektierte Referenzlicht 102 ungebeugt durch die Transmissions-Beugungsgitteranordnung 36 und trifft auf die Austrittsfläche 33 des ersten Beugungselements 3 wo es von einem ersten Array-Detektor 37, z.B. einer Kamera erfasst wird.

Auf der anderen Seite des Beugungselements 3, also der auf Seite der zweiten Eintrittsfläche 32, wird Laserlicht in Form von Objektlicht 200 mittels einer Abbildungsoptik 12 (vgl. Fig. 5 oder 6) des Systems 1 auf ein zu erfassendes Objekt 300 (vgl. Fig. 5 oder 6) projiziert. Das Objektlicht 200 wird dabei in Form eines Intensitätsmusters auf das Objekt 300 projiziert, wobei das Intensitätsmuster (nicht dargestellt) aus einer Vielzahl an lateral versetzten und lateral nicht überlappenden Spots, also einer Vielzahl an beleuchteten Bereichen, die durch nicht beleuchtete Bereiche voneinander getrennt sind, besteht.

Objektlicht 200 aus einem beleuchteten Bereich des Intensitätsmusters wird durch das Objekt 300 in Richtung der Holografie-Anordnung 2 zurückreflektiert oder zurückgestreut. Um das Objektlicht 200 einzusammeln, weist das System 1 eine Objektivlinse 6 auf. Im erfindungsgemäßen Betrieb, wird die Objektivlinse 6 in einem Abstand zum Intensitätsmuster gehalten, der in etwa der Brennweite der Objektivlinse 6 entspricht. Auf diese Weise wird das Objektlicht 200 des Intensitätsmusters kollimiert. Die perfekte Kollimation setzt den Objektbereich im Fokus der Objektivlinse 6 voraus. Bereiche von außerhalb der Brennebene, d.h. in einem unterschiedlichen z-Abstand, würden ein leicht gekrümmtes Wellenfeld generieren. Die mathematische Betrachtung eines solchen Wellenfeldes ist dem Fachmann bekannt. Daher wird in der Hauptsache das Bild über die Strahlungsvektoren bemüht.

Das kollimierte Objektlicht 200 trifft auf die zweite Eintrittsfläche 32 des ersten Beugungselements 3 (oder wenn das System zwei Beugungselemente 3, 3' aufweist, auf die zweite Eintrittsfläche 32' des zweiten Beugungselements 3', siehe z.B. Fig. 6) und zwar so, dass es auf die Transmissions-Beugungsgitteranordnung 36, 36' im Beugungselement 3, 3' annährend in einem Winkel von 45° trifft, also in Littrow-Konfiguration. Dazu kann die Objektivlinse 6 je nach Konfiguration des Beugungselements 3, 3' eine bestimmte Position und Ausrichtung relativ zur optischen Achse OA des Beugungselements 3, 3' haben - wenn beispielweise die zweite Eintrittsfläche 32, 32' des Beugungselements 3, 3' einen von 90° verschiedenen Winkel mit der optische Achse OA des Beugungselements 3, 3' einnimmt, so sollte die optische Achse der Objektivlinse 6 ggf. in einem Winkel zur optischen Achse OA des Beugungselements 3, 3' angeordnet sein, so dass das kollimierte Objektlicht 200 nach Eintritt in das Beugungselement 3, 3' entlang der optischen Achse OA des Beugungselements 3, 3' propagiert, so dass die Littrow-Konfiguration sichergestellt ist. Die Transmissions-Beugungsgitteranordnung 36 beugt sodann das Objektlicht 201 in Richtung Austrittsfläche 33 wo es mit dem gebeugten und reflektierten Referenzlicht 102 überlagert wird und ggf. ein Interferenzmuster ausbildet, das vom ersten Array-Detektor 37 erfasst wird. Aus den Interferenzmustern kann die 3D-Information des Objektes 300 in bekannter Weise errechnet werden kann.

Der erste Array-Detektor 37 kann entlang seiner Detektionsebene 370 in einem von 45° verschiedenen Winkel ε = 45° ± η zur Beugungsebene 360 angeordnet sein. Dies kann in vorteilhafter Weise Rückreflexionen unterdrücken.

In der allgemeinen Variante der Holografie-Anordnung 2, 2' (wie beispielsweis ein Fig. 1 dargestellt), können die Mantelflächenbereiche des ersten Beugungselements 3 von 45° verschiedene Winkel zur Beugungsebene 360 einschließen. Dies kann ebenfalls vorteilhafte Auswirkungen bzgl. störender Rückreflexionen von Oberflächen haben. Insbesondere sind die folgenden Winkel in Fig. 1 dargestellt:
Winkel ω₁, der von der ersten Eintrittsfläche und der Beugungsebene eingeschlossen wird, wobei ω₁ im Bereich von 30° und 60° liegen kann.
Winkel ω₂, der von der Prismafläche und der Beugungsebene eingeschlossen wird. Dabei wird eine optionale Winkelabweichung von 45° des Winkels ω₂ durch den Winkel ρ₂ beschrieben.
Winkel ω₃, der von der zweiten Eintrittsfläche und der Beugungsebene eingeschlossen wird, wobei ω₃ im Bereich von 30° und 60° liegen kann.
Winkel ω₄, der von der Austrittsfläche und der Beugungsebene eingeschlossen wird, wobei ω₄ im Bereich von 30° und 60° liegen kann.
Winkel α, der von dem ersten Spiegel und der Beugungsebene eingeschlossen wird. Dabei wird eine optionale Winkelabweichung von 45° des Winkels α durch den Winkel β beschrieben.
Winkel ε, der von dem ersten Array-Detektor, genauer mit der Detektionsebene des Array-Detektors und der Beugungsebene eingeschlossen wird. Dabei wird eine optionale Winkelabweichung von 45° durch den Winkel η beschrieben.

Wie bereits eingangs erwähnt, können in analoger Weise die Winkelbezeichnungen, die aufgezeigten Winkelverhältnisse zwischen den Flächen und Ebenen der ersten Holografie-Anordnung 2, sowie die Funktionsweise (z.B. Littrow-Anordnung) der ersten Holografie-Anordnung 2 auf eine zweite Holografie-Anordnung 2' übertragen werden. D.h. beispielsweise ist anstelle des ersten Beugungselements 3 dann ein zweites Beugungselement 3' vorhanden, anstelle des ersten Spiegels 35 ist ein zweiter Spiegel 35' Teil der zweiten Holografie-Anordnung 2' und anstelle eines ersten Array-Detektors 37 ist ein zweiter Array-Detektor 37' auf Seite der Austrittsfläche 33' des zweiten Beugungselements 3' angeordnet. Aus diesem Grund sind in Fig. 1 auch die entsprechenden Winkelbezeichnungen und Bezugszeichen für die Komponenten der zweiten Holografie-Anordnung 2' entsprechend dargestellt, für die auch bzgl. der Winkel und Funktionsweise im Wesentlichen das Gleiche wie für die erste Holografie-Anordnung 2 gilt. Im Detail bedeutet dies:
Winkel ω'₁ wird von der ersten Eintrittsfläche 31' bzw. Eintrittsebene 310' und der Beugungsebene 360' des zweiten Beugungselements 3' eingeschlossen, wobei ω'₁ im Bereich von 30° und 60° liegen kann.
Winkel ω'₂ wird von der Prismafläche 34' und der Beugungsebene 360' des zweiten Beugungselements 3' eingeschlossen. Dabei wird eine optionale Winkelabweichung von 45° des Winkels ω'₂ durch den Winkel ρ'₂ beschrieben.
Winkel ω'₃ wird von der zweiten Eintrittsfläche 32' und der Beugungsebene 360' des zweiten Beugungselements 3' eingeschlossen, wobei ω'₃ im Bereich von 30° und 60° liegen kann.
Winkel ω'₄ wird von der Austrittsfläche 33' und der Beugungsebene 360' des zweiten Beugungselements 3' eingeschlossen, wobei ω'₄ im Bereich von 30° und 60° liegen kann.
Winkel α' wird von dem zweiten Spiegel 35' und der Beugungsebene 360' des zweiten Beugungselements 3' eingeschlossen. Dabei wird eine optionale Winkelabweichung von 45° des Winkels α' durch den Winkel β' beschrieben.
Winkel ε' wird von dem zweiten Array-Detektor 37', genauer mit der Detektionsebene 370' des zweiten Array-Detektors 37' und der Beugungsebene 360' des zweiten Beugungselements 3' eingeschlossen. Dabei wird eine optionale Winkelabweichung von 45° durch den Winkel η' beschrieben.

Es ist allerdings festzuhalten, dass es vorteilhaft ist, sofern eine zweite Holografie-Anordnung 2' im System 1 umfasst ist, dass die erste Eintrittsfläche 31' des zweiten Beugungselements 3' parallel zur zweiten Eintrittsfläche 32 des ersten Beugungselements 3 verläuft, so dass die beiden Eintrittsflächen 32, 31' miteinander insbesondere ohne Ausbildung eine Luftspalts verbunden sein können, so dass die Beugungsebenen der Transmissions-Beugungsgitteranordnung 36, 36' des ersten und des zweiten Beugungselements 3, 3' entweder parallel zueinander verlaufen (vgl. z.B. Fig. 6) oder einen 90° Winkel (nicht dargestellt) miteinander einschließen. Insbesondere ist vorgesehen, dass bei einer Ausführungsform mit zwei Holografie-Anordnungen 2, 2' die erste Eintrittsfläche 31' des zweiten Beugungselements 3' und die zweite Eintrittsfläche 32 des ersten Beugungselements 3 jeweils einen Winkel von 45° mit der jeweiligen Beugungsebene 360, 360' einnehmen, also senkrecht zur jeweiligen optischen Achse OA des Beugungselements 3, 3' stehen (vgl. Fig. 6).

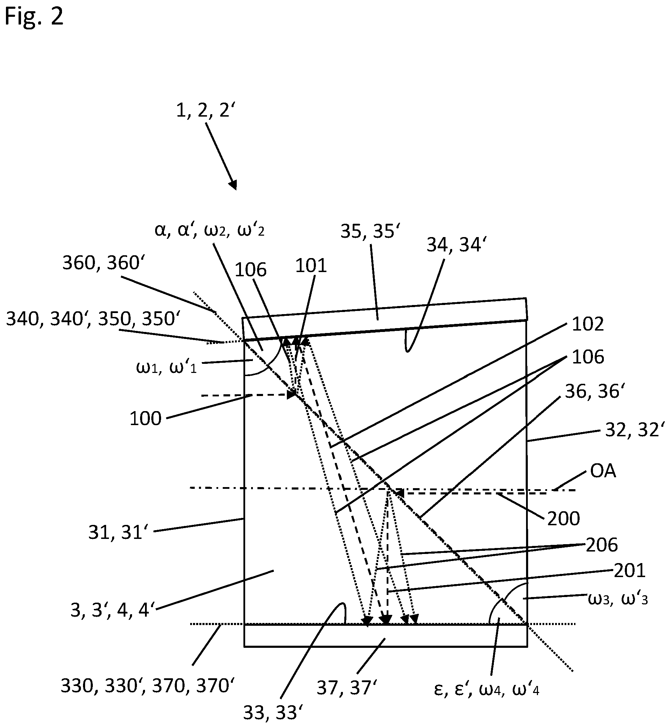

Zum Laserlicht und dem daraus hervorgehenden Referenzlicht 100 und Objektlicht 200 ist weiterhin folgendes angemerkt. Die Erfindung sieht insbesondere vor, dass das Laserlicht eine Vielzahl an Wellenlängen umfasst. Besonders vorteilhaft ist es dabei, wenn in einem Wellenlängenbereich um die Zentralwellenlänge 100-1, 100-2 bzw. 200-1, 200-2, eine Vielzahl an separaten spektralen Linien 106 bzw. 206 (vgl. z.B. Figs. 2 und 5) z.B. Laserlinien im Laserlicht umfasst sind. Eine spektrale Line hat beispielsweise eine Breite im Bereich von bis 0.5 nm, vorzugsweise kleiner als 0.1 nm. Der Wellenlängenbereich wiederum mag eine typische Breite von bis etwa 10 nm aufweisen. Das Laserlicht und die Transmissions-Beugungsgitteranordnung 36, 36' des Systems 1 sind insbesondere aufeinander abgestimmt. Da das Transmissions-Beugungsgitteranordnung 36, 36' mindestens ein Volumen-Phasen-Hologramm Gitter umfasst, kann zunächst folgendes angemerkt werden. Das Volumen-Phasen-Hologramm Gitter weist eine Bandbreite um eine Zentralwellenlänge idealerweise die Zentralwellenlänge des Laserlichts auf, innerhalb derer es in ausreichendem Maße in der Littrow-Konfiguration einfallendes Licht entsprechend beugt, wobei die Zentralwellenlänge "perfekt" um 2*45° = 90° gebeugt wird.

Laser mit leicht unterschiedlichen Wellenlängen um eine Zentralwellenlänge sind als Frequenzkamm-Laser bekannt und können z.B. mit der Quantum-Dot Technologie hergestellt werden. Eingesetzt werden diese z.B. für Frequency-Multiplexing einer Breitband-Übertragung in der Telekommunikation. Alternativ ist auch denkbar, dass single-mode stripe Laser mit leicht unterschiedlichen Wellenlängen dicht an dicht auf einem Laserdioden-Modul integriert sind und die Ausgangs-Aperturen über ein speziell integriert-optisches Chip zu einem Laserlichtquellen-Modul kombiniert werden. Auch ein Fasersplitter könnte Teil solch eines Modules zu werden. Evtl. ist solch eine Kombination bereits auf dem Laserdioden-Chip selbst vorstellbar, was den Herstellungs-Prozess vereinfachen würde. Vereinfacht soll von einer Laserlichtquelle mit einer Zentralwellenlänge gesprochen werden.

Multiple Wellenlängen um die Zentralwellenlänge sind vorteilhaft oder gar notwendig, um über die Phasen der einzelnen Wellenlängensignale pro Objektpunkt bzw. pro Beleuchtungs-Spot eine eindeutige Tiefeninformation zu erhalten. Die lateralen Objektinformationen werden aus den Hologramm-Frequenzen z.B. durch Fouriertransformation erzeugt. In Kombination wird so ein 3D-Objekt prinzipiell erkannt, was dem Fachmann im Prinzip bekannt ist.

Im Folgenden wird näher auf die vorteilhaften Eigenschaften eines Volumen-Phasen-Hologramm Gitters eingegangen:
Bei Auftreffen von Referenz oder Objektlicht auf das Volumen-Phasen-Hologramm Gitter mit der Gitterfrequenz *f_{gr}* wird das Licht mit der Zentralwellenlänge *λ₀* in der Littrow-Anordnung um exakt 90° (= 2*45°) in Richtung der Prismafläche 34, 34' bzw. der Austrittsfläche 33, 33' wie bereits geschildert gebeugt. Bei leicht variierender Wellenlänge (multiple Linien im Wellenlängenbereich des Laserlichts) ergibt sich in dieser Littrow-Anordnung nachfolgende Winkelaufspaltung Δ*θ*: $\frac{Δθ}{Δλ} = \frac{{f}_{gr}}{cos \left(45°\right)} = \frac{2}{λ 0}$ Gl. (1)

Bei der Zentralwellenlänge 660 nm sind das 3 mrad/ nm und bei einer Zentralwellenlänge von 1300 nm sind das 1.5 mrad/ nm. Beträgt ein typischer Abstand zwischen zwei spektralen Linien 200 pm, so verändert sich der Winkel damit um Δ*θ* = 0.6 mrad. Sofern die Wellenlängenänderung positiv (λ₀ + Δλ) ist, erhöht sich der Beugungswinkel auf 90° + Δ*θ*.

Für Objekt- und Referenzlicht gilt, dass das Volumen-Phasen-Hologramm Gitter Referenz- und Objektlicht jeweils in die entgegengesetzte Richtung beugt, also den doppelten Winkel gegenüber Gl. (1) liefert, also 2*Δ*θ*.

Der Vorteil des Volumen-Hologramms gegenüber einem rein planaren Gitter liegt in der Selektivität bzgl. Winkel und Spektrum. Beide Eigenschaften werden in bereits beschriebener Weise genutzt.

Bei Betrachtung des obigen Beispiels wären daher 2*0.6 mrad = 1.2 mrad für eine Wellenlängenänderung von 200 pm zu veranschlagen. Bei typischen Auflösungsgrenzen von 0.1 mrad pro Pixel auf dem Array-Detektor bedeutet dies, dass mindestens 10 Pixel Versatz zu einer benachbarten spektralen Linie mit der nächst höheren/ niedrigeren Wellenlänge existiert. Die Brechzahl der Prisma-Anordnung verstärkt obigen Winkel nochmals um den Faktor *n_{Prisma}* bei Austritt Richtung Array-Detektor, da die Strahlen vom Lot weg gebeugt werden. Da die Brechzahlen sich typischer Weise zwischen 1.5 - 1.9 bewegen, ist mit einer entsprechenden Zunahme zu rechnen.

Aus diesen Überlegungen wird klar, dass das die Transmissions-Beugungsgitteranordnung mit einem Volumen-Phasen-Hologramm Gitter in Littrow-Konfiguration in der vorgeschlagenen Prisma-Anordnung bereits besonders vorteilhafte Eigenschaften zur spektralen Separierung einer Vielzahl an spektralen Linien aufweist. Es ist angemerkt, dass die in Gl. (1) behandelte Winkelaufspaltung lediglich aus der Dispersion des Volumen-Phasen-Hologramm Gitter stammt und nicht aus dem Winkel α oder ω₂.

Der Winkel α bzw. der Winkel ω₂ bewirkt, dass gebeugtes und am ersten Spiegel reflektiertes Referenzlicht unter einem Winkel auf die Transmissions-Beugungsgitteranordnung trifft, in der das Referenzlicht zum großen Teil (insbesondere >70%) nicht mehr gebeugt wird, sondern in Richtung der Austrittsfläche weiterpropagiert und dadurch eine ausreichend hohe Intensität an Referenzlicht an der Austrittsfläche zur Verfügung steht, um mit dem Objektlicht ein Interferenzmuster auszubilden. Der entsprechende Strahlverlauf ist schematisch in Fig. 1 angedeutet. Die gleichen Überlegungen gelten für eine zweite Holografie-Anordnung des Systems.

Für die folgende Betrachtung wird zu Illustrationszwecken angenommen, dass der erste Spiegel 35 auf der Prismafläche 34 angeordnet ist und damit die Winkel α und ω₂ identisch sind.

In diesem Fall trifft das in Littrow-Konfiguration eingestrahlte und vom ersten Spiegel reflektierte Referenzlicht der Zentralwellenlänge λ₀ in einem Winkel 45°± 2β, auf die Beugungsebene und weist demnach gegenüber dem gebeugten Objektlicht einen Winkel von 2β auf. Der wellenlängenverschobene Anteil λ₀ ± Δλ des reflektierten Referenzlichts weist gegenüber dem entsprechenden wellenlängenverschobenen Anteil λ₀ ± Δλ des gebeugten Objektlichtes einen Winkel von 2Δ*θ*+2β auf. Die verschiedenen Winkel 2Δ*θ* bewirken entsprechende Hologramm-Frequenzen auf dem Kamerachip, welche durch Fouriertransformation den multiplen Wellenlängen zugeordnet werden können. Die Anzahl dieser Wellenlängen kann minimal zwei, aber auch mehrere Duzend betragen. Da moderne Kamerachips mehrere tausend Pixel pro Dimension haben können, sind noch genügend Raumfrequenzen (Winkel) frei um die lateralen Bildpunkte über die Fouriertransformation im Objekt zuordnen zu können, was dem Fachmann an sich bekannt ist. Sofern der Array-Detektor über einen Luftspalt zur Austrittsfläche des Beugungselements beabstandet ist, werden die Winkel noch einmal wie bereits oben erläutert um den Faktor des Brechungsindex der Prisma-Anordnung verstärkt.

Der Neigungswinkel β kann in etwa wie folgt aus der Art Disperionsbeziehung eines Volumen-Phasen-Hologramm Gitters mit Zentralwellenlänge λ₀, dem Brechungsindex n (~1,5) des Gitters sowie der Dicke *D* des Volumen-Phasen-Hologramm Gitters hergeleitet werden: ${δ}_{FWHM} \approx \frac{n {λ}_{0}}{D}$ Gl. (2)

Wobei δ_{FWHM} derjenige Einstrahlwinkel ist, bei dem die Intensität des gebeugten Lichtes nur noch ca. 50% beträgt. Bei einer Dicke des Volumen-Phasen-Hologramm Gitters von *D* = 10 µm ergibt sich bei einer Wellenlänge λ₀ = 660 nm und einem Brechungsindex n = 1.5 ein Winkel δ_{FWHM}/2 ≈ 50 mrad, nach der Definition der FWHM ("Full Width at Half Maximum"). Damit sollte das von dem ersten Spiegel reflektierte Referenzlicht, das unter einem Winkel von 2β auf die Beugungsebene trifft die Bedingung erfüllen, also δ_{FWHM}/2 ≈ 2β ≈ 50 mrad, so dass das Referenzlicht zur Hälfte ungebeugt durch die Transmissions-Beugungsgitteranordnung propagiert, während die andere Hälfte wieder in Richtung der ersten Eintrittsfläche weggebeugt wird. Da das Referenzlicht in der Holografie aber immer deutlich stärker als der Objektstrahl ist, stellt die Abschwächung des Referenzstrahls um den Faktor 2 keine Einschränkung dar.

Der Einsatz eines Volumen-Phasen-Hologramm Gitter hat demnach den weiteren Vorteil, dass das Objektlicht innerhalb der FWHM zu einem großen Teil Richtung Austrittsfläche gebeugt wird. Bei einem "klassischen" Gitter kann dies zwar auch erreicht werden, aber das reflektierte Referenzlicht würde zu einem großen Teil in Richtung der ersten Eintrittsfläche weggebeugt und würde für die Interferenzbildung an der Austrittsfläche nur mit schwacher Signalstärke zur Verfügung stehen. Daher müsste in diesem Fall die Beugungseffizienz des Beugungsgitters (z.B. auf 50%) herabgesetzt werden, was dann wiederum zu einer Abschwächung des gebeugten Objektlichts in Richtung Austrittsfläche führen würde.

Ein weiterer Vorteil von Volumen-Phasen-Hologramm Gittern ist, dass es möglich ist, zwei Volumen-Phasen-Hologramm Gitter mit unterschiedlicher Zentralwellenlänge ineinander angeordnet zu erzeugen, oder alternativ zwei dieser vergleichsweise dünnen Volumen-Phasen-Hologramm Gitter Strukturen aufeinander anzuordnen.

In den Figuren 2 bis 6 werden exemplarisch einige besonders vorteilhafte Ausführungsformen des optischen Systems 1 erläutert. Gleiche Bezugszeichen bezeichnen die gleichen Elemente, Komponenten oder Merkmale.

In Fig. 2 ist eine vorteilhafte Ausführungsform einer ersten oder zweiten Holografie-Anordnung 2, 2' gemäß der Erfindung dargestellt.

Bezugnehmend auf die eingeführten Winkelbeziehungen und Bezugszeichen liegen die Winkel ω₁, ω₃, ω₄ (bzw. sofern es sich um ein zweites Beugungselement handelt, die Winkel ω'₁, ω'₃, ω'₄) des Beugungselements bei 45°.

Weiterhin sind die Winkel ω₂ und β (bzw. ω'₂ und β') identisch und der erste bzw. der zweite Spiegel 35, 35' ist auf der Prismafläche 34, 34' des Beugungselements 3, 3' in Form einer reflektierenden Schicht ausgebildet. Dies hat den Vorteil, dass ein robustes und kompaktes optisches System 1 geschaffen wird. Weiterhin ermöglicht diese Konfiguration der ersten bzw. der zweiten Holografie-Anordnung 2, 2' eine serielle Anordnung in einem optischen System gemäß der Erfindung (vgl. Fig. 6), bei der das erste und das zweite Beugungselement 3, 3' mit den jeweiligen Eintrittsflächen 32, 31' nebeneinander ohne Luftspalt angeordnet werden können.

Der erste bzw. zweite Array-Detektor 37, 37' ist ebenso direkt auf der Austrittsfläche 33, 33' des Beugungselements 3, 3' fixiert, so dass auch hier kein Luftspalt entsteht.

Insbesondere kann die Prisma-Anordnung 4, 4' in Fig. 2 einstückig oder aus zwei entlang der Beugungsebene 360, 360',miteinander über die Transmissions-Beugungsgitteranordnung 36, 36' verbundenen Dreiecksprismen geformt sein.

Im Fig. 2 ist nun beispielhaft der Strahlengang des Referenzlichts im Beugungselement 3bzw. 3' für Referenzlicht, das um eine Zentralwellenlänge zwei weitere spektrale Linien 106 umfasst, die beispielsweise die Randwerte des Wellenlängenbereichs um die Zentralwellenlänge des Laserlichts 100, 200 und des darauf angepassten Volumen-Phasen-Hologramm Gitters bilden. Ausgehen von Referenzlicht 100 werden die spektralen Linien durch die Transmissions-Beugungsgitteranordnung 36, bzw. 36 weniger oder mehr als die Zentralwellenlänge gebeugt. Daher schließen die Strahlen der spektralen Linien 106 nachdem sie durch die Transmissions-Beugungsgitteranordnung 36, 36' gebeugt wurden einen Winkel ungleich 45° mit dem der Beugungsebene ein. Durch die Winkelstellung α, α' des Spiegels 35, 35', divergieren die reflektierten spektralen Linien weiter vom Strahl 102 um den Strahl der Zentralwellenlänge 102 und treffen an lateral unterschiedlichen Orten auf die Austrittsfläche 33, 33'.

Gleiches gilt für das Objektlicht 200, das durch die Transmissions-Beugungsgitteranordnung 36, 36' gebeugt wird. Die spektralen Linien 206 des Laserlichts führen auch hier zu einer dispersiven Aufspaltung des Objektlichts 200 und in verschieden stark gebeugte spektrale Linien 206 durch die Transmissions-Beugungsgitteranordnung 36, 36'.

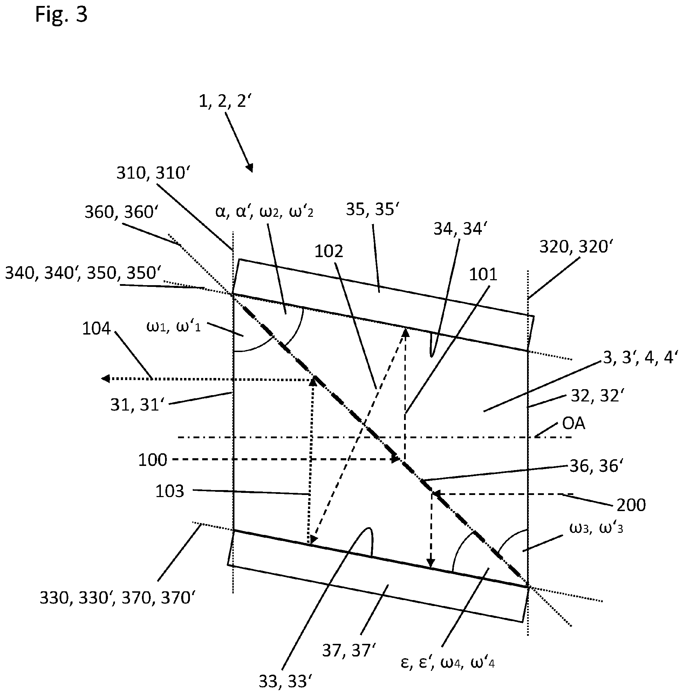

In Fig. 3 ist eine Variante der ersten oder zweiten Holografie-Anordnung 2, 2' des optischen Systems 1 zu sehen, bei der die Prisma-Anordnung 4, 4' eine Grundfläche in Form eines Parallelogramms aufweist, wobei die erste und zweite Eintrittsfläche 31, 32, (bzw. 31', 32') jeweils einen Winkel ω₁, ω₃ (bzw. ω'₁, ω'₃) von 45° mit der Beugungsebene 360, 360' einschließen - also insbesondere senkrecht zur optischen Achse OA des Beugungselements 3, 3' stehen, und die Austrittsfläche 33, 33' sowie die Prismafläche 34, 34' ebenfalls parallel verlaufen, so dass die Winkel ω₂ und ω₄ (bzw. ω'₂ und ω'₄) identisch sind und kleiner 45°. Ebenso ist in diesem Ausführungsbeispiel der Spiegel 35, 35' direkt (also ohne einen Spalt auszubilden) auf der Prismafläche 34, 34' angeordnet, so dass der Winkel α gleich dem Winkel ω₂ ist (und damit β = ρ₂) bzw. der Winkel α' gleich dem Winkel ω'₂ ist (und damit β' = ρ'₂). Der Spiegel 35, 35' kann wiederum als reflektierende Schicht ausgebildet sein, die mit einem entsprechenden Verfahren beispielsweise auf die Prismafläche 34, 34' aufgedampft wurde.

Darüber hinaus ist der Array-Detektor 37, 37' direkt auf der Austrittsfläche 33, 33' angeordnet, sodass auch die Winkel ε und ω₄ (bzw. ε' und ω'₄) identisch sind. Somit ergeben sich folgende Winkelrelationen: $α = {ω}_{2} = {ω}_{4} = ε < 45 ° bzw . α ′ = ω {′}_{2} = ω {′}_{4} = ε ′ < 45 °$ oder, $α = {ω}_{2} = {ω}_{4} = ε > 45 ° bzw . α ′ = ω {′}_{2} = ω {′}_{4} = ε ′ > 45 . °$

Diese Ausführungsform besticht durch eine einfache Geometrie die gleichzeitig eine Unterdrückung von Störsignalen (Ghosting) auf dem Array-Detektor 37, 37' verbessert, da wie durch die gepunkteten Pfeile 103 und 104 angedeutet, potenziell vom Array-Detektor 37, 37' reflektiertes Licht aufgrund der besonderen Winkelbeziehungen der Flächen der Holografie-Anordnung 2, 2', dieses Licht wieder in einem Winkel von 45° auf die Transmissions-Beugungsgitteranordnung 36, 36' trifft und - bei hoher Beugungseffizienz - wieder in Richtung der ersten Eintrittsfläche 31, 31' gebeugt und damit aus dem Beugungselement 3, 3' herausgeleitet wird.

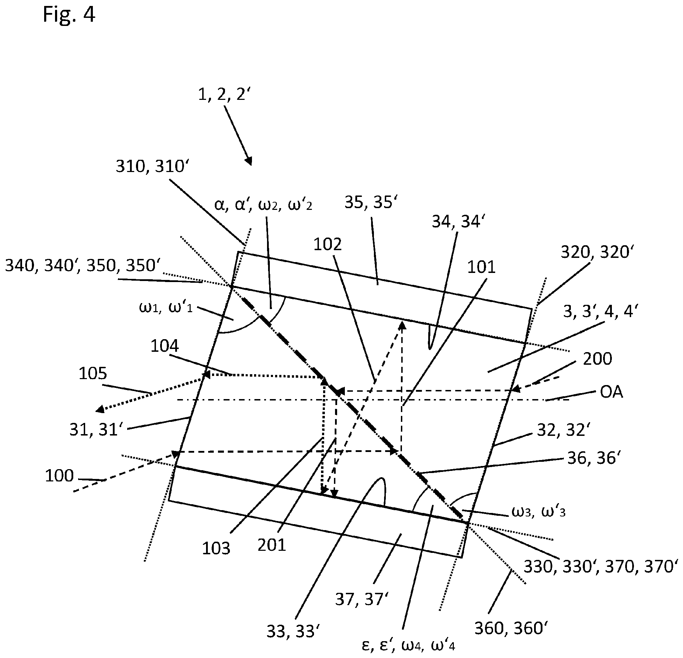

Ähnlich zu Fig. 3 zeigt Fig. 4 eine erweiterte Variante der ersten oder zweiten Holografie-Anordnung 2, 2'. Auch hier weist die Prisma-Anordnung 4, 4' ein Parallelogramm als Grundfläche auf, wobei im Unterschied zu Fig. 3 auch die Winkel der ersten und zweiten Eintrittsfläche ω₁ und ω₃ (bzw. ω'₁ und ω'₃) ungleich 45° sind.

Dadurch ist gewährleistet, dass die Eintrittsflächen 31, 32, (bzw. 31', 32') nicht senkrecht zur optischen Achse OA des Beugungselements 3, 3'verlaufen, was eine vorteilhafte Ablenkung der an diesen Flächen potenziell auftretenden Rückreflexen (siehe Pfeil 105) zeigt.

Es ist anzumerken, dass auch diese Geometrie in einem System 1 umfassend zwei in Serie angeordnete Holografie-Anordnungen 2, 2' zum Einsatz kommen kann, indem die erste Eintrittsfläche 31' des zweiten Beugungselements 3' ohne Luftspalt an der zweiten Eintrittsfläche 32 des ersten Beugungselements 3 angeordnet wird.

In Fig. 5 ist ein optisches System 1 gemäß eine Ausführungsform der Erfindung dargestellt, wobei zusätzlich zu der Holografie-Anordnung 2, 2' aus den vorherigen Abbildungen nun auch weitere Komponenten des Systems 1 schematisch dargestellt sind.

Zunächst umfasst das System in Fig. 5 eine Laserlichtquelle 8 (gepunktete Box) mit lediglich einem Laser 81, die dazu eingerichtet ist, Laserlicht um eine Zentralwellenlänge und ggf. mit weiteren spektralen Linien innerhalb eines Wellenlängenbereichs zur Verfügung zu stellen. Die dafür geeigneten Laserlichtquellen bzw. Laser sind bereits in vorhergehenden Absätzen beispielhaft offenbart worden. Die Laserlichtquelle 8 kann darüber hinaus dazu eingerichtet sein, polarisiertes Laserlicht einer bestimmten Polarisation zu emittieren. Das Laserlicht wird sodann mittels eines Strahlteilers in Referenzlicht 100 und Objektlicht 200 geteilt. Das Teilungsverhältnis kann dabei ungleich 50:50 sein, wobei bevorzugt ein größerer Anteil z.B. 70% oder 90% des Laserlichtes dem Objektlicht zugeteilt wird.

Der Strahlteiler kann in einem Fasersplitter 13 integriert sein, der dazu eingerichtet ist, das Referenzlicht 100 in eine erste polarisationserhaltende single-mode Faser 10 einzukoppeln und das Objektlicht 200 in eine zweite polarisationserhaltende single-mode Faser 11 einzukoppeln. Die erste Faser 9 wird mit einem Ende in einem Brennpunkt oder zumindest in einer Brennebene der Kollimationsoptik 5 des optischen Systems 1 angeordnet, so dass Referenzlicht 100 ggf. mittels einer entsprechenden Auskopplungslinse oder Apertur 10 aus der Faser 9 auskoppelt wird und die Wellenfront des Referenzlichtes von der Kollimationsoptik 5 in kollimiert wird. Die Kollimationsoptik 5 weist eine optische Achse auf, die im Beispiel der Fig. 5 kolinear mit der optischen Achse OA des ersten Beugungselements 3 verläuft. Wenn die erste Eintrittsfläche 31 mit der Beugungsebene 360 einen Winkel ungleich 45° einnimmt, dann sollte die optische Achse der Kollimationsoptik 5 entsprechend ausgerichtet sein, damit das Referenzlicht nach Eintritt in das Beugungselement 3 entlang der optischen Achse OA des Beugungselements 3 propagiert. In diesem Beispiel ist die optische Achse der Kollimationsoptik 5 parallel zur optischen Achse des ersten Beugungselements 3 verschoben, so dass Referenzlicht in einem Bereich näher zum Spiegel in das Beugungselement 3 eintritt und damit mit den entsprechenden Strahlen 201, 206 des Objektlichtes am Array-Detektor 37 überlagert wird. Der weitere Propagationsverlauf in der ersten Holografie-Anordnung 2 wurde bereits erschöpfend behandelt und wird hier nicht erneut wiedergegeben.

Das Objektlicht 200 propagiert nach dem Fasersplitter 13 entlang der zweiten optischen Faser 11 und wird durch eine Abbildungsoptik 12 am Ende der zweiten Faser 11 emittiert und zwar so, dass ein Intensitätsmuster auf ein zu vermessendes Objekt 300 projiziert wird.

Das Intensitätsmuster weist auf dem Objekt 300 mindestens einen Beleuchtungs-Spot, bevorzugt aber viele Spots auf, die durch nicht beleuchtete Bereiche des Musters voneinander lateral getrennt sind.

Das vom Objekt 300 zurückgeworfene Objektlicht 200 aus dem Muster wird von einer Objektivlinse 6 des Systems 1 erfasst und, wenn es aus der Brennebene der Objektivlinse 6 stammt, kollimiert in Richtung der zweiten Eintrittsfläche 32 geleitet. Dort tritt das Objektlicht 200 in das Beugungselement 3 ein und wird in Richtung Austrittsfläche 33 gebeugt, wo es zu Überlagerung mit dem Referenzlicht 102 kommt. Diese Überlagerung wird von der ersten Array-Detektor 37 aufgezeichnet und zur weiteren Verarbeitung beispielsweise in Form von digitalen Daten an einen Computer 400 weitergeleitet. Am Computer 400 kann eine Rekonstruktion des Wellenfeldes des Objektlichtes vorgenommen werden, so dass eine 3D-Represenation der beleuchteten Bereiche des Objekts erzeugt werden kann.

Die lateral im Intensitätsmuster versetzten Spots führen dazu, dass jeder Spot auf dem ersten Array-Detektor 37 trotz der spektralen Aufspaltung auf dem Array-Detektor 37 noch einzeln analysiert werden kann. Um eine vollständige Information über das Objekt zu erhalten, sollte das Objekt mit dem Intensitätsmuster abgetastet werden, so dass idealerweise jeder Teil des Objekts zumindest einmal mit einem Spot beleuchtet und von dem ersten Array-Detektor 37 erfasst wurde.

Wie bereits in Fig. 2 dargestellt ist beispielhaft in Fig. 5 ebenfalls der Strahlengang für die spektralen Linien 106 bzw. 206 aus dem Wellenlängenbereich um die Zentralwellenlänge der Referenzlichtstrahlen 102 und Objektlichtstrahlen 201 dargestellt, der durch die Dispersionseigenschaften der Transmissions-Beugungsgitteranordnung 36 bzw. des Volumen-Phasen-Hologramm Gitter vorteilhaft bedingt ist.

Die in Fig. 5 dargestellte Holografie-Anordnung kann ohne Beeinträchtigung des Systems 1 durch eine andere zuvor offenbarte Holografie-Anordnung 2 ersetzt werden, wobei ggf. entsprechen die Position und Orientierung der Kollimationsoptik 5 und/oder der Objektivlinse 6 angepasst werden muss, damit das System 1 die Littrow-Konfiguration einnimmt.

In Fig. 6 ist eine Ausführungsform des Systems 1 aus Fig. 5 dargestellt, das im Unterschied zum System in Fig. 5 eine erste und eine zweite Holografie-Anordnung 2, 2' umfasst. Die erste Holografie-Anordnung 2 umfasst ein erstes Volumen-Phasen-Hologramm Gitter 36, das für eine erste Zentralwellenlänge 100-1, 200-1 eingerichtet ist, und die zweite Holografie-Anordnung 2' umfasst ein Volumen-Phasen-Hologramm Gitter 36', das für eine zweite Zentralwellenlänge 100-2, 200-2 eingerichtet ist. D.h. das Volumen-Phasen-Hologramm Gitter 36 des ersten Beugungselements 3 beugt in der Littrow-Konfiguration das Referenz- und Objektlicht um die erste Zentralwellenlänge 100-1, 200-1 und lässt Licht um die zweite Zentralwellenlänge 100-2 ungebeugt passieren, währen das Volumen-Phasen-Hologramm Gitter 36' des zweiten Beugungselements 3' in der Littrow-Konfiguration das Referenz- und Objektlicht um die zweite Zentralwellenlänge 100-2, 200-2 beugt und Licht um die erste Zentralwellenlänge 200-1ungebeugt passieren lässt. Dadurch kann ein vergrößerter Spektralbereich zur digitalen Holografie verwendet werden.

Es ist angemerkt, dass in Fig. 6 aus Gründen der Übersichtlichkeit die Strahlengänge der spektralen Linien 106 und 206 um die jeweiligen Zentralwellenlängen 100-1, 100-2, 200-1, 200-2 nicht dargestellt sind.

Um Laserlicht mit den zwei Zentralwellenlängen zur Verfügung zu stellen umfasst die Laserlichtquelle 8 zwei Laser 81, 82, wobei ein erster Laser 81 dazu eingerichtet ist, Laserlicht um die erste Zentralwellenlänge 100-1, 200-1 zu emittierten (und insbesondere weitere spektrale Linien in einem Wellenlängenbereich um die erste Zentralwellenlänge), und wobei ein zweiter Laser 82 dazu eingerichtet ist, Laserlicht um die zweite Zentralwellenlänge 100-2, 200-2 zu emittierten (und insbesondere weitere spektrale Linien in einem Wellenlängenbereich um die zweite Zentralwellenlänge). Das Licht der beiden Laser 81, 82 wird durch einen Fasersplitter 13 in die erste und zweite polarisationserhaltende Faser 9, 11 eingekoppelt, wie bereits zu Fig. 5 beschrieben. Sofern die Bandbreite der Fasern 9, 11 nicht ausreicht beide Zentralwellenlängen single-mode zu transportieren, können auch jeweils separate Fasern für jede Zentralwellenlänge verwendet werden, die ggf. an Ihren Enden über einen Strahlteiler kombiniert werden oder leicht lateral versetzt angeordnet sind, bevor Sie über die Abbildungsoptik 12 auf das Objekt 300 projiziert werden bzw. über die Kollimationsoptik 5 kollimiert werden.

Im Beispiel der Fig. 6 sind das erste und das zweite Beugungselement 3, 3' in Serie angeordnet und die zweite Eintrittsfläche 32 des ersten Beugungselements 3 ist mit der ersten Eintrittsfläche 31' des zweiten Beugungselements 3' luftspaltfrei verbunden, so dass insbesondere ein Sprung im Brechungsindex beim Übergang zwischen den Beugungselementen 3, 3' vermieden wird. Dazu verlaufen die beiden Eintrittsflächen 31', 32 parallel zueinander. Je nach Anforderung, schließen der erste und der zweite Spiegel 35, 35' den gleichen oder einen unterschiedlichen Winkel α, α' mit der jeweiligen Beugungsebene 360, 360' ein.

Jedes Beugungselement 3,3' umfasst einen eigenen Array-Detektor 37, 37', die ggf. einen optischen Filter (nicht dargestellt) vorgeschaltet haben, um Streulicht oder Reflexionen aus anderen Wellenlängenbereichen außer dem vorgesehenen zu blockieren.

Die von dem ersten und zweiten Array-Detektor 37, 37' aufgezeichneten Signale an der jeweiligen Austrittsfläche 33, 33' werden demselben Computer 400 zugeführt, der aus den Informationen eine 3D-Repräsentation des Objekts errechnen kann.

Das erfindungsgemäße System 1 erlaubt es in kompakter und robuster Weise digitale Holografie z.B. im minimal-invasiven Medizinbereich zu ermöglichen. Das System ist insbesondere dazu eingerichtet und geeignet in einem Endoskop umfasst zu sein.

| | | | |
|---|---|---|---|
| Bezugszeichenliste | | 35 | erster Spiegel |
| 1 | optisches System | 36, 36' | Transmissions-Beugungsgitteranordnung |
| 2 | erste Holografie-Anordnung | 37 | erster Array-Detektor |
| 2' | zweite Holografie- | 37' | zweiter Array-Detektor |
| | Anordnung | OA | optische Achse des Beugungslements |
| 3 | erstes Beugungslement | | |
| 3' | zweites Beugungslement | ω1 | Winkel zwischen 310 und 360 |
| 4 | erste Prisma-Anordnung | ω'1 | Winkel zwischen 310' und 360' |
| 4' | zweite Prisma-Anordnung | | |
| 5 | Kollimationsoptik | ω2 | Winkel zwischen 340 und 360 |
| 6 | Objektivlinse | ω'2 | Winkel zwischen 340' und 360' |
| 8 | Laserllichtquelle | | |
| 81, 82 | Laser | ω3 | Winkel zwischen 320 und 360 |
| 9 | erste optische Faser | | |
| 10 | Eingangsapertur | ω'3 | Winkel zwischen 320' und 360' |
| 11 | zweite optische Faser | ω4 | Winkel zwischen 330 und 360 |
| 12 | Abbildungsoptik | | |
| 31, 31' | erste Eintrittsfläche | ω'4 | Winkel zwischen 330' und 360' |
| 32, 32' | zweite Eintrittsfläche | | |
| 33, 33' | Austrittsfläche | α | Winkel zwischen 350 und 360 |
| 34, 34' | Prismafläche | | |
| α' | Winkel zwischen 350' und 360' | 200-1 | erste Zentralwellenlänge Objektlicht |
| ε | Winkel zwischen 370 und 360 | 200-2 | zweite Zentralwellenlänge Objektlicht |
| ε' | Winkel zwischen 370' und 360' | 300 | Objekt |
| | | 400 | Computer |
| X | Winkel zwischen 102 und 360 | | |
| 100 | Referenzlicht | | |
| 101 | gebeugtes Referenzlicht | | |
| 102 | reflektiertes Referenzlicht | | |
| 103-105 | Lichtreflektionen Referenzlicht | | |
| 106 | spektrale Linien um Zentrawellenlänge Referenzlicht | | |
| 100-1 | erste Zentralwellenlänge Referenzlicht | | |
| 100-2 | zweite Zentralwellenlänge Referenzlicht | | |
| 200 | Objektlicht | | |
| 201 | gebeugtes Objektlicht | | |
| 206 | spektrale Linien um Zentrawellelänge Objektlicht | | |

## Patentansprüche

1. Ein optisches System (1) zur digitalen Holografie, umfassend zumindest die folgenden Komponenten:
eine erste Holografie-Anordnung (2), umfassend ein erstes Beugungselement (3), welches durch eine erste Prisma-Anordnung (4) mit zumindest viereckiger Grundfläche gebildet ist, wobei eine Mantelfläche der ersten Prisma-Anordnung (4) die folgenden Mantelflächenbereiche aufweist:
a) eine erste Eintrittsfläche (31) für Referenzlicht (100), die sich entlang einer ersten Eintrittsebene (310) erstreckt,
b) eine zweite Eintrittsfläche (32) für Objektlicht (200), die sich entlang einer zweiten Eintrittsebene (320) erstreckt, wobei die erste und zweite Eintrittsflächen (31, 32) gegenüberliegende Mantelflächenbereiche der ersten Prisma-Anordnung (4) ausbilden,
c) eine Austrittsfläche (33), die sich entlang einer Austrittebene (330) erstreckt und durch die gebeugtes Referenzlicht (102) und gebeugtes Objektlicht (201) aus dem ersten Beugungselement (3) austreten kann,
d) eine der Austrittsfläche (33) gegenüberliegende Prismafläche (34), die sich entlang einer Prismaebene (340) erstreckt,
e) eine optische Transmissions-Beugungsgitteranordnung (36), die in dem ersten Beugungselement (3) angeordnet ist, und sich entlang einer Beugungsebene (360), welche die erste Eintrittsebene (310) schneidet, zwischen der ersten Eintrittsfläche (31) und der Austrittsfläche (33) erstreckt,
**dadurch gekennzeichnet, dass** die Transmissions-Beugungsgitteranordnung (36) des ersten Beugungselements (3) zumindest ein erstes Volumen-Phasen-Hologramm Gitter umfasst, und dass die erste Holografie-Anordnung (2) auf Seite der Prismafläche (34) einen ersten Spiegel (35) mit einer ersten Spiegelebene (350) aufweist, wobei die erste Spiegelebene (350) mit der Beugungsebene (360) einen Winkel α einschließt und die Prismaebene (340) mit der Beugungsebene (360) einen Winkel ω₂ einschließt, wobei zumindest einer der Winkel α, ω₂ von 45° verschieden ist, wobei das System (1) einen ersten Array-Detektor (37) umfasst, wobei der erste Array-Detektor (37) dazu eingerichtet ist, aus der Austrittsfläche (33) des ersten Beugungselements (3) austretendes Licht zu detektieren.

2. Das optische System (1) nach Anspruch 1, **dadurch gekennzeichnet, dass** der erste Array-Detektor (37) an der Austrittsfläche (33) des ersten Beugungselements (3) angeordnet ist.

3. Das optische System (1) nach einem der vorhergehenden Ansprüche, **dadurch gekennzeichnet, dass** die Grundfläche der ersten Prisma-Anordnung (4) ein Parallelogramm und insbesondere ein Rechteck ist.

4. Das optische System (1) nach einem der vorhergehenden Ansprüche, **dadurch gekennzeichnet, dass** der erste Spiegel (37) auf der Prismafläche (33) des ersten Beugungselements (3) angeordnet oder ausgebildet ist.

5. Das optische System (1) gemäß einem der vorhergehenden Ansprüche, **dadurch gekennzeichnet, dass** die optische Transmissions-Beugungsgitteranordnung (36) der ersten Holografie-Anordnung ein zweites Volumen-Phasen-Hologramm Gitter umfasst, wobei das erste und das zweite Volumen-Phasen-Hologramm Gitter verschiedene Zentralwellenlängen aufweisen.

6. Das optische System (1) nach einem der vorhergehenden Ansprüche, umfassend eine zweite Holografie-Anordnung (2') mit einem zweiten Beugungselement (3'), welches durch eine zweite Prisma-Anordnung (4') mit zumindest viereckiger Grundfläche gebildet ist, wobei die erste und die zweite Holografie-Anordnung (2, 2') nebeneinander entlang einer optischen Achse (OA) des ersten Beugungselements (3) angeordnet sind, und eine Mantelfläche der zweiten Prisma-Anordnung (4') die folgenden Mantelflächenbereiche aufweist:
f) eine erste Eintrittsfläche (31') für Referenzlicht (100), die sich entlang einer ersten Eintrittsebene (310') erstreckt,
g) eine zweite Eintrittsfläche (32') für Objektlicht (200), die sich entlang einer zweiten Eintrittsebene (320') erstreckt, wobei die erste und zweite Eintrittsfläche (31', 32') gegenüberliegende Mantelflächenbereiche der zweiten Prisma-Anordnung (4') ausbilden,
h) eine Austrittsfläche (33'), die sich entlang einer Austrittebene (330') erstreckt und durch die gebeugtes Referenzlicht (102) und gebeugtes Objektlicht (201) aus dem zweiten Beugungselement (3') austreten kann,
i) eine der Austrittsfläche (33') gegenüberliegende Prismafläche (33'), die sich entlang einer Prismaebene (330') erstreckt,
j) eine optische Transmissions-Beugungsgitteranordnung (36'), die im zweiten Beugungselement (3') angeordnet ist und sich entlang einer Beugungsebene (360'), welche die erste Eintrittsebene (310') schneidet, zwischen der ersten Eintrittsfläche (31') und der Austrittsfläche (33') des zweiten Beugungselements (3') erstreckt,
**dadurch gekennzeichnet, dass** die Transmissions-Beugungsgitteranordnung (36') des zweiten Beugungselements (3') zumindest ein erstes Volumen-Phasen-Hologramm Gitter umfasst, und dass die zweite Holografie-Anordnung (2') auf Seite der Prismaebene (340') einen zweiten Spiegel (35') mit einer zweiten Spiegelebene (350') aufweist, wobei die zweite Spiegelebene (350') mit der Beugungsebene (360') einen Winkel α' einschließt und die Prismaebene (340') mit der Beugungsebene (360') einen Winkel ω'₂ einschließt, wobei zumindest einer der Winkel α', ω'₂ von 45° verschieden ist.

7. Das optische System (1) gemäß Anspruch 6, **dadurch gekennzeichnet, dass** die zweite Eintrittsfläche (32) des ersten Beugungselements (3) mit der ersten Eintrittsfläche (31') des zweiten Beugungselements (3') verbunden ist.

8. Das optische System (1) gemäß einem der vorhergehenden Ansprüche, wobei das optische System (1) auf Seite der ersten Eintrittsfläche (31) des ersten Beugungselements (3) eine Kollimationsoptik (5) für Referenzlicht (100) aufweist, die eine optische Achse aufweist, welche in Richtung der ersten Eintrittsfläche (31) des ersten Beugungselements (3) verläuft und wobei die Kollimationsoptik (5) dazu eingerichtet ist, Referenzlicht (100) vor Eintritt durch die erste Eintrittsfläche (31) des ersten Beugungselements (3) zu kollimieren.

9. Das optische System (1) gemäß einem der vorhergehenden Ansprüche, wobei das optische System (2) eine Objektivlinse (6) für Objektlicht (200) umfasst, wobei die Objektivlinse (6) so im optischen System (1) angeordnet ist, dass von der Objektivlinse (6) in Richtung zweiter Eintrittsfläche (32, 32') des ersten und/oder des zweiten Beugungselements (3, 3') propagierendes Objektlicht (200) kollimiert ist, wenn das Objektlicht (200) in und/oder nahe einer Brennebene der Objektivlinse (6) in Richtung der Objektivlinse (6) abstrahlt.

10. Das optische System (1) gemäß einem der vorhergehenden Ansprüche, **dadurch gekennzeichnet, dass** das optische System (1) die folgenden Komponenten umfasst:
- eine Laserlichtquelle (8), die dazu eingerichtet ist, Laserlicht mit einer oder mehreren Zentralwellenlängen zu Verfügung zu stellen,
- zumindest eine erste optische Faser (9), die dazu eingerichtet ist, das Laserlicht der Laserlichtquelle (8) zu einer Eingangsapertur (10) der Kollimationsoptik (5) zu leiten, so dass die Kollimationsoptik (5) das Laserlicht kollimiert in Form von Referenzlicht (100) zur ersten Holografie-Anordnung (2) leitet,
- zumindest eine zweite optische Faser (11), die dazu eingerichtet ist, das Laserlicht der Laserlichtquelle (8) zu einer Ausgangsapertur (12) des optischen Systems (1) zu leiten, von der aus ein zu erfassendes Objekt (300) mit Laserlicht in Form von Objektlicht (200) beleuchtet wird.

11. Das optische System (1) gemäß Anspruch 10, **dadurch gekennzeichnet, dass** das optische System (1) einen Fasersplitter (13) umfasst, der dazu ausgebildet ist, das Laserlicht der Laserlichtquelle (8) zu teilen und in die erste und zweite optische Faser (9, 11) einzukoppeln.

12. Das optische System nach Anspruch 11, wobei der Fasersplitter einen polarisationserhaltenden Fasersplitter umfasst, wobei die erste und die zweite optische Faser jeweils polarisationserhaltende single-mode Fasern sind.

13. Das optische System (1) gemäß einem der vorhergehenden Ansprüche **dadurch gekennzeichnet, dass** das optische System (1) eine Abbildungsoptik (12) aufweist, die dazu eingerichtet ist, Objektlicht (200) in Form eines Intensitätsmusters auf ein zu erfassendes Objekt (300) zu projizieren, wobei das Intensitätsmuster aus zumindest einem beleuchteten Bereich besteht.

14. Das System nach Anspruch 13, wobei das Intensitätsmuster aus einer Vielzahl an disjunkten beleuchteten Bereichen besteht, wobei die beleuchteten Bereiche des Intensitätsmusters punkt- oder kreisförmig sind.

15. Das optische System (1) gemäß einem der vorhergehenden Ansprüche, **dadurch gekennzeichnet, dass** das optische System (1) dazu eingerichtet ist, Laserlicht zur Verfügung zu stellen, welches Wellenlängen aus zumindest zwei Wellenlängenbereichen umfasst, wobei ein erster Wellenlängenbereich um eine erste Zentralwellenlänge (100-1, 200-1) gelagert ist und Wellenlängen insbesondere in Form von spektralen Linien umfasst, die außerhalb eines zweiten Wellenlängenbereichs liegen, wobei der zweite Wellenlängenbereich um eine zweite Zentralwellenlänge (100-2, 200-2) gelagert ist und Wellenlängen insbesondere in Form spektralen Linien umfasst, wobei insbesondere der erste und der zweite Wellenlängenbereich jeweils einen spektralen Bereich von nicht mehr als 50 nm umfassen, insbesondere nicht mehr als 15 nm, insbesondere wobei die spektralen Linien der Wellenlängenbereiche jeweils eine Linienbreite von nicht mehr als 0.5 nm aufweisen.

## Claims

1. An optical system (1) for digital holography, comprising at least the following components:
a first holographic assembly (2), comprising a first diffraction element (3) formed by a first prism assembly (4) with an at least quadrangular base, wherein a lateral surface of the first prism assembly (4) comprises the following lateral surface regions:
a) a first incident surface (31) for reference light (100), which extends along a first incident plane (310)
b) a second incident surface (32) for object light (200), which extends along a second incident plane (320), wherein the first and second incident surfaces (31, 32) form opposing lateral surface regions of the first prism assembly (4),
c) an exit surface (33) extending along an exit plane (330), through which diffracted reference light (102) and diffracted object light (201) can emerge from the first diffraction element (3),
d) a prism surface (34) opposite the exit surface (33), which extends along a prism plane (340),
e) an optical transmission diffraction grid assembly (36) which is arranged within the first diffraction element (3) and extends along a diffraction plane (360), which intersects the first incident plane (310), between the first incident surface (31) and the exit surface (33),
**characterised in that** the transmission diffraction grid assembly (36) of the first diffraction element (3) comprises at least one first volume-phase hologram grid, and **in that** the first holographic assembly (2) comprises, on the side of the prism surface (34), a first mirror (35) with a first mirror plane (350) on the side of the prism surface (34), wherein the first mirror plane (350) forms an angle α with the diffraction plane (360) and the prism plane (340) forms an angle ω2 with the diffraction plane (360), wherein at least one of the angles α, ω2 is different from 45°, wherein the system (1) comprises a first array detector (37), the first array detector (37) being configured to detect light emerging from the exit surface (33) of the first diffraction element (3).

2. The optical system (1) according to claim 1, **characterised in that** the first array detector (37) is arranged at the exit surface (33) of the first diffraction element (3).

3. The optical system (1) according to one of the preceding claims, **characterised in that** the base area of the first prism assembly (4) is a parallelogram and, in particular, a rectangle.

4. The optical system (1) according to one of the preceding claims, **characterised in that** the first mirror (37) is arranged on or formed on the prism face (33) of the first diffraction element (3).

5. The optical system (1) according to one of the preceding claims, **characterised in that** the optical transmission diffraction grid assembly (36) of the first holographic assembly comprises a second volume-phase hologram grid, wherein the first and second volume-phase hologram grids have different central wavelengths.

6. The optical system (1) according to one of the preceding claims, comprising a second holographic assembly (2') with a second diffraction element (3'), which is formed by a second prism assembly (4') having an at least quadrangular base, wherein the first and second holographic assemblies (2, 2') are arranged side by side along an optical axis (OA) of the first diffraction element (3), and a lateral surface of the second prism assembly (4') comprises the following lateral surface regions:
f) a first incident surface (31') for reference light (100), which extends along a first incident plane (310'),
g) a second incident surface (32') for object light (200), which extends along a second incident plane (320'), wherein the first and second incident surfaces (31', 32') form opposing lateral surface regions of the second prism assembly (4'),
h) an exit surface (33') extending along an exit plane (330'), through which diffracted reference light (102) and diffracted object light (201) can emerge from the second diffraction element (3'),
i) a prism surface (33') opposite the exit surface (33'), which extends along a prism plane (330'),
j) an optical transmission diffraction grid assembly (36') which is arranged within the second diffraction element (3') and extends along a diffraction plane (360'), which intersects the first entry plane (310'), between the first entry surface (31') and the exit surface (33') of the second diffraction element (3'),
**characterised in that** the transmission diffraction grid assembly (36') of the second diffraction element (3') comprises at least a first volume-phase hologram grid, and **in that** the second holographic assembly (2') comprises, on the side of the prism plane (340'), a second mirror (35') with a second mirror plane (350'), wherein the second mirror plane (350') forms an angle α' with the diffraction plane (360') and the prism plane (340') forms an angle ω'₂ with the diffraction plane (360'), wherein at least one of the angles α', ω'₂ is different from 45°.

7. The optical system (1) according to claim 6, **characterised in that** the second incident surface (32) of the first diffractive element (3) is connected to the first incident surface (31') of the second diffractive element (3').

8. The optical system (1) according to one of the preceding claims, wherein the optical system (1) comprises, on the side of the first incident surface (31) of the first diffraction element (3), a collimating optical system (5) for reference light (100), which has an optical axis extending in the direction of the first incident surface (31) of the first diffraction element (3), and wherein the collimating optics (5) are configured to collimate reference light (100) prior to its entry through the first incident surface (31) of the first diffraction element (3).

9. The optical system (1) according to one of the preceding claims, wherein the optical system (2) comprises an objective lens (6) for object light (200), the objective lens (6) being arranged in the optical system (1) such that object light (200) propagating from the objective lens (6) towards a second incident surface (32, 32') of the first and/or second diffraction element (3, 3') is collimated when the object light (200) radiates in the direction of the objective lens (6) within and/or near a focal plane of the objective lens (6).

10. The optical system (1) according to one of the preceding claims, **characterised in that** the optical system (1) comprises the following components:
- a laser light source (8) configured to provide laser light with one or more central wavelengths,
- at least one first optical fibre (9), which is configured to guide the laser light from the laser light source (8) to an input aperture (10) of the collimating optics (5), such that the collimating optics (5) guide the laser light, collimated in the form of reference light (100), to the first holographic assembly (2),
- at least one second optical fibre (11), which is arranged to guide the laser light from the laser light source (8) to an exit aperture (12) of the optical system (1), from which an object (300) to be detected is illuminated with laser light in the form of object light (200).

11. The optical system (1) according to claim 10, **characterised in that** the optical system (1) comprises a fibre splitter (13) configured to split the laser light from the laser light source (8) and couple it into the first and second optical fibres (9, 11).

12. The optical system according to claim 11, wherein the fibre splitter comprises a polarisation-maintaining fibre splitter, and wherein the first and second optical fibres are each polarisation-maintaining single-mode fibres.

13. The optical system (1) according to any one of the preceding claims, **characterised in that** the optical system (1) comprises a projection optics (12) which is configured to project object light (200) in the form of an intensity pattern onto an object (300) to be detected, wherein the intensity pattern consists of at least one illuminated area.

14. The system according to claim 13, wherein the intensity pattern consists of a plurality of disjoint illuminated regions, wherein the illuminated regions of the intensity pattern are point-shaped or circular.

15. The optical system (1) according to any one of the preceding claims, **characterised in that** the optical system (1) is configured to provide laser light comprising wavelengths from at least two wavelength ranges, wherein a first wavelength range centres on a first central wavelength (100-1, 200-1) and comprises wavelengths, in particular in the form of spectral lines, which lie outside a second wavelength range, wherein the second wavelength range is centred on a second central wavelength (100-2, 200-2) and comprises wavelengths, in particular in the form of spectral lines, wherein, in particular, the first and second wavelength ranges each comprise a spectral range of no more than 50 nm, in particular no more than 15 nm, and in particular the spectral lines of the wavelength ranges each have a line width of no more than 0.5 nm.

## Revendications

1. Système optique (1) pour l'holographie numérique, comprenant au moins les composants suivants :
un premier agencement d'holographie (2), comprenant un premier élément de diffraction (3) qui est formé par un premier agencement de prisme (4) avec au moins une face de base quadrangulaire, dans lequel une face d'enveloppe du premier agencement de prisme (4) présente les régions de face d'enveloppe suivantes :
a) une première face d'entrée (31) pour de la lumière de référence (100) qui s'étend le long d'un premier plan d'entrée (310),
b) une seconde face d'entrée (32) pour de la lumière d'objet (200) qui s'étend le long d'un second plan d'entrée (320), dans lequel les première et seconde faces d'entrée (31, 32) forment des régions de face d'enveloppe opposées du premier agencement de prisme (4),
c) une face de sortie (33) qui s'étend le long d'un plan de sortie (330) et à travers laquelle de la lumière de référence diffractée (102) et de la lumière d'objet diffractée (201) peuvent sortir du premier élément de diffraction (3),
d) une face de prisme (34) opposée à la face de sortie (33) qui s'étend le long d'un plan de prisme (340),
e) un agencement de réseau de diffraction de transmission optique (36) qui est disposé dans le premier élément de diffraction (3) et s'étend le long d'un plan de diffraction (360), qui coupe le premier plan d'entrée (310), entre la première face d'entrée (31) et la face de sortie (33),
**caractérisé en ce que** l'agencement de réseau de diffraction de transmission optique (36) du premier élément de diffraction (3) comprend au moins un premier réseau d'hologramme de phase volumique, et que le premier agencement d'holographie (2) présente sur le côté de la face de prisme (34) un premier miroir (35) avec un premier plan de miroir (350), dans lequel le premier plan de miroir (350) inclut un angle α avec le plan de diffraction (360) et le plan de prisme (340) inclut un angle ω₂ avec le plan de diffraction (360),
dans lequel au moins un des angles α, ω₂ est différent de 45°, dans lequel le système (1) comprend un premier détecteur de matrice (37), dans lequel le premier détecteur de matrice (37) est configuré pour détecter de la lumière sortant de la face de sortie (33) du premier élément de diffraction (3).

2. Système optique (1) selon la revendication 1, **caractérisé en ce que** le premier détecteur de matrice (37) est disposé sur la face de sortie (33) du premier élément de diffraction (3).

3. Système optique (1) selon une des revendications précédentes, **caractérisé en ce que** la face de base du premier agencement de prisme (4) est un parallélogramme et notamment un rectangle.

4. Système optique (1) selon une des revendications précédentes, **caractérisé en ce que** le premier miroir (37) est disposé ou réalisé sur la face de prisme (33) du premier élément de diffraction (3).

5. Système optique (1) selon une des revendications précédentes, **caractérisé en ce que** l'agencement de réseau de diffraction de transmission optique (36) du premier agencement d'holographie comprend un second réseau d'hologramme de phase volumique, dans lequel le premier et le second réseau d'hologramme de phase volumique présentent différentes longueurs d'onde centrales.

6. Système optique (1) selon une des revendications précédentes, comprenant un second agencement d'holographie (2') avec un second élément de diffraction (3') qui est formé par un second agencement de prisme (4') avec au moins une face de base quadrangulaire, dans lequel le premier et le second agencement d'holographie (2, 2') sont disposés côte à côte le long d'un axe optique (OA) du premier élément de diffraction (3), et une face d'enveloppe du second agencement de prisme (4') présente les régions de face d'enveloppe suivantes :
f) une première face d'entrée (31') pour de la lumière de référence (100) qui s'étend le long d'un premier plan d'entrée (310'),
g) une seconde face d'entrée (32') pour de la lumière d'objet (200) qui s'étend le long d'un second plan d'entrée (320'), dans lequel les première et seconde faces d'entrée (31', 32') forment des régions de face d'enveloppe opposées du second agencement de prisme (4'),
h) une face de sortie (33') qui s'étend le long d'un plan de sortie (330') et à travers laquelle de la lumière de référence diffractée (102) et de la lumière d'objet diffractée (201) peuvent sortir du second élément de diffraction (3'),
i) une face de prisme (33') opposée à la face de sortie (33') qui s'étend le long d'un plan de prisme (330'),
j) un agencement de réseau de diffraction de transmission optique (36') qui est disposé dans le second élément de diffraction (3') et s'étend le long d'un plan de diffraction (360'), qui coupe le premier plan d'entrée (310'), entre la première face d'entrée (31') et la face de sortie (33') du second élément de diffraction (3'),
**caractérisé en ce que** l'agencement de réseau de diffraction de transmission optique (36') du second élément de diffraction (3') comprend au moins un premier réseau d'hologramme de phase volumique, et que le second agencement d'holographie (2') présente sur le côté du plan de prisme (340') un second miroir (35') avec un second plan de miroir (350'), dans lequel le second plan de miroir (350') inclut un angle α' avec le plan de diffraction (360') et le plan de prisme (340') inclut un angle ω'₂ avec le plan de diffraction (360'), dans lequel au moins un des angles α', ω'₂ est différent de 45°.

7. Système optique (1) selon la revendication 6, **caractérisé en ce que** la seconde face d'entrée (32) du premier élément de diffraction (3) est connectée à la première face d'entrée (31') du second élément de diffraction (3').

8. Système optique (1) selon une des revendications précédentes, dans lequel le système optique (1) présente sur le côté de la première face d'entrée (31) du premier élément de diffraction (3) une optique de collimation (5) pour de la lumière de référence (100) qui présente un axe optique qui s'étend en direction de la première face d'entrée (31) du premier élément de diffraction (3) et dans lequel l'optique de collimation (5) est configurée pour collimater de la lumière de référence (100) avant l'entrée à travers la première face d'entrée (31) du premier élément de diffraction (3).

9. Système optique (1) selon une des revendications précédentes, dans lequel le système optique (1) comprend une lentille de focalisation (6) pour de la lumière d'objet (200), dans lequel la lentille de focalisation (6) est disposée dans le système optique (1) de sorte que de la lumière d'objet (200) se propageant de la lentille de focalisation (6) en direction de la seconde face d'entrée (32, 32') du premier et/ou du second élément de diffraction (3, 3') est collimatée lorsque la lumière d'objet (200) irradie dans et/ou à proximité d'un plan focal de la lentille de focalisation (6) en direction de la lentille de focalisation (6).

10. Système optique (1) selon une des revendications précédentes, **caractérisé en ce que** le système optique (1) comprend les composants suivants :
- une source de lumière laser (8) qui est configurée pour mettre à disposition de la lumière laser avec une ou plusieurs longueurs d'onde centrales,
- au moins une première fibre optique (9) qui est configurée pour guider la lumière laser de la source de lumière laser (8) vers une ouverture d'entrée (10) de l'optique de collimation (5) de sorte que l'optique de collimation (5) guide la lumière laser collimatée sous forme de lumière de référence (100) vers le premier agencement d'holographie (2),
- au moins une seconde fibre optique (11) qui est configurée pour guider la lumière laser de la source de lumière laser (8) vers une ouverture de sortie (12) du système optique (1) de laquelle un objet à détecter (300) est éclairé avec de la lumière laser sous forme de lumière d'objet (200).

11. Système optique (1) selon la revendication 10, **caractérisé en ce que** le système optique (1) comprend un séparateur de fibre (13) qui est réalisé pour diviser la lumière laser de la source de lumière laser (8) et l'injecter dans la première et seconde fibre optique (9, 11).

12. Système optique selon la revendication 11, dans lequel le séparateur de fibre comprend un séparateur de fibre à obtention de polarisation, dans lequel la première et la seconde fibre optique sont chacune des fibres monomodales à obtention de polarisation.

13. Système optique (1) selon une des revendications précédentes, **caractérisé en ce que** le système optique (1) présente une optique de représentation (12) qui est configurée pour projeter de la lumière d'objet (200) sous forme d'un modèle d'intensité sur un objet à détecter (300), dans lequel le modèle d'intensité se compose d'au moins une région éclairée.

14. Système optique selon la revendication 13, dans lequel le modèle d'intensité se compose d'une pluralité de régions éclairées disjointes, dans lequel les régions éclairées du modèle d'intensité sont en forme de point ou de cercle.

15. Système optique (1) selon une des revendications précédentes, **caractérisé en ce que** le système optique (1) est configuré pour mettre à disposition de la lumière laser qui comprend des longueurs d'onde provenant d'au moins deux plages de longueur d'onde, dans lequel une première plage de longueur d'onde est incorporée autour d'une première longueur d'onde centrale (100-1, 200-1) et comprend des longueurs d'onde notamment sous forme de lignes spectrales qui se situent en dehors d'une seconde plage de longueur d'onde, dans lequel la seconde plage de longueur d'onde est incorporée autour d'une seconde longueur d'onde centrale (100-2, 200-2) et comprend des longueurs d'onde notamment sous forme de lignes spectrales, dans lequel la première et la seconde plage de longueur d'onde comprennent notamment chacune une région spectrale de pas plus de 50 nm, notamment pas plus de 15 nm, notamment dans lequel les lignes spectrales des plages de longueur d'onde présentent chacune une largeur de ligne de pas plus de 0,5 nm.
